# EUROPEAN PATENT SPECIFICATION

(11) **EP 3 283 081 B1**
(45) Date of publication and mention of the grant of the patent: **03.07.2019**
(21) Application number: 16720367.8
(22) Date of filing: 15.04.2016
(51) Int. Cl.: A61K 31/795, A61K 33/44, C01B 32/194, B82Y 30/00, B82Y 40/00, C08K 3/04, G01N 33/569, A61P 31/12

(54) **GRAPHENE DERIVATIVE AND METHODS FOR MANUFACTURING THE SAME**
GRAPHENDERIVAT UND VERFAHREN ZUR HERSTELLUNG DAVON
DÉRIVÉ DE GRAPHÈNE ET PROCÉDÉS POUR LE FABRIQUER

(30) Priority: 15.04.2015 DE 102015206814
(43) Date of publication of application: 21.02.2018
(73) Proprietor: Freie Universität Berlin, 14195 Berlin (DE)
(72) Inventor: HAAG, Rainer, 12209 Berlin (DE); ZIEM, Benjamin, 13507 Berlin (DE); NITSCHE, Andreas, 14165 Berlin (DE)
(74) Representative: Maikowski & Ninnemann Patentanwälte Partnerschaft mbB
(86) International application number: PCT/EP2016/058392
(87) International publication number: WO 2016/166316

(56) References cited:
- MATIAS SAMETBAND ET AL: "Herpes Simplex Virus Type-1 Attachment Inhibition by Functionalized Graphene Oxide", ACS APPLIED MATERIALS AND INTERFACES, vol. 6, no. 2, 22 January 2014 (2014-01-22), pages 1228-1235, XP055286979, US ISSN: 1944-8244, DOI: 10.1021/am405040z
- JONATHAN VONNEMANN ET AL: "Virus inhibition induced by polyvalent nanoparticles of different sizes", NANOSCALE, vol. 6, no. 4, 1 January 2014 (2014-01-01) , page 2353, XP055286991, United Kingdom ISSN: 2040-3364, DOI: 10.1039/c3nr04449a
- PHAM T A ET AL: "Covalent functionalization of graphene oxide with polyglycerol and their use as templates for anchoring magnetic nanoparticles", SYNTHETIC METALS, ELSEVIER SEQUOIA, LAUSANNE, CH, vol. 160, no. 17-18, 1 September 2010 (2010-09-01), pages 2028-2036, XP027259358, ISSN: 0379-6779 [retrieved on 2010-08-19]

## Description

The instant invention relates to a graphene derivative according to the preamble of claim 1, to the in vitro use of such a graphene derivative according to the preamble of claim 5, to the use of such a graphene derivative as medicament according to the preamble of claim 6, to an anti-viral medicament according to the preamble of claim 8, to uses of the graphene derivative according to the preambles of claims 9 to 13, and to two methods of manufacturing such a graphene derivative according to the preambles of claims 14 and 15.

The market for antiviral therapeutics has increased over the years which also caused an increase in using antiviral drugs for therapies. But the careless behavior of the human being in case of handling these drugs led to rapid and widespread development of drug resistance. Some of the "old" antiviral drugs (e.g., amantadine and rimantadine for influenza virus infections) have lost already their clinical utility [1]. Because of this increased resistance it is indispensable on the one hand to develop new drugs and novel therapies and on the other hand a general treatment strategy for the existing antiviral drugs would be useful. One of the still effective drugs is the anticoagulant drug heparin, which was established by the work of Crafoord and Best for prevention of postoperative thrombosis in the 1930s [2]. However, the application of heparin for antiviral treatment bares a high risk of undesirable side effects, such as bleeding complications or heparin-induced thromboxytopenia [3].

JP 2014-172781 A describes surface-modified graphene as well as a method of manufacturing the same. This Japanese patent application relates in particular to graphene derivatives being additionally acylated with a fatty acid residue.

DE 10 2006 036 326 A1 describes dendritic polyglycerol sulfates that can optionally be immobilized by a matrix.

MATIAS SAMETBAND ET AL: "Herpes Simplex Virus Type-1 Attachment Inhibition by Functionalized Graphene Oxide",ACS APPLIED MATERIALS AND INTERFACES, vol. 6, no. 2, (2014), pages 1228-1235, discloses sulfonated graphene oxide having antiviral activity.

It is an object of the instant invention to provide a compound that can be used, amongst others, as anti-viral drug as well as methods for manufacturing this compound.

This object is achieved by a graphene derivative having the features of claim 1.

Such a graphene derivative or compound comprises at least one graphene layer. This graphene layer can be substituted or non-substituted. Several graphene layers one above the other build up graphite. Thus, the graphene derivative or compound can also be denoted as graphite derivative or compound if it contains more than one graphene layer. According to the invention, the graphene layer is functionalized with a covalently bound polyglycerol compound comprising a polyglycerol backbone and at least one substituent in the nature of a covalently bound sulfate group. Also disclosed are any negatively charged group chosen from the group consisting of sulfonates, phosphates, phosphonates, bisphosphonates, carboxylates and combinations thereof.

Single layer graphene is a slightly wrinkled, honeycomb-like monolayer sheet based on a two-dimensional (2D) arrangement of sp²-hybridized carbon atoms. It exhibits unique physical properties as it is the strongest (130 GPa) ever measured material. Additionally, it provides a high thermal as well as a very high electrical conductivity and, compared to other chemical compounds, offers a huge surface area of approximately 2600 m²/g, available for chemical modifications.

The graphene derivative can be denoted as a two-dimensional (2D) hybrid architecture, providing a huge surface functionalized with polyglycerol compounds for specific interaction with viral particles.

In an embodiment, the substituent is a sulfate. Thus, in this embodiment, the polyglycerol compound is a polyglycerol sulfate.

In an embodiment, a degree of substitution of the backbone is between 10 and 100 % (including the upper and lower limit). In an embodiment, the degree of substitution of the backbone is between 15 % and 95 %, in particular between 20 % and 90 %, in particular between 25 % and 85 %, in particular between 30 % and 80 %, in particular between 35 % and 75 %, in particular between 40 % and 70 %, in particular between 45 % and 65 %, in particular between 50 % and 60 %, in particular between 55 % and 58 %, (in each case including the upper and lower limits). A very well suited range of substitution is between 70 % and 100 % (including the upper and lower limit).

The degree of substitution, in particular the degree of sulfation, can be adjusted by adjusting the experimental conditions under which the substitution takes place. Generally, the higher the degree of substitution with negatively charged substituents, the higher is the molecular interaction with viruses. This molecular interaction takes place on the basis of ionic interactions, wherein multivalent effects are observed and can be exploited.

In an embodiment, the graphene layer is additionally substituted by hydroxyl groups. Such a hydroxyl group bearing graphene can be produced the following way:
In a first step, graphene is oxidized to graphite oxide (GO) by the Hummers-Offeman oxidation [4, 5] in which different functional groups are inserted into the structure of the honeycomb-like sp²-carbon material resulting in primarily epoxy and hydroxyl groups on top and on the bottom of the GO sheets and carboxyl groups at the edges (Lerf-Klinowski model).

This insertion ruptures the carbon lattice and leads to a formation of smaller fragments as well as an increase of the interlayer distance. Then, monolayer or few-layer graphene can be obtained by an exfoliation of the graphite oxide. Through the exfoliation the volume of the thermally reduced graphene oxide (TRGO) increases 100 to 300 fold. About 80% of the generated graphene sheets can be considered as monolayers with an average size of 500 nm in their longest extension [16].

The adoption of a polyglycerol compound as covalent binding partner can cause defects or even rupture the honeycomb-like lattice of the graphene. However, the insertion of functional groups improves the solubility, processability and opens a new range of applications.

The disposal of the polyglycerol compound to the carbon material will not only lead to an enhanced solubility especially for water but also to a multifunctional surface which is accessable for further postmodification like sulfation or other targeting moieties (in particular negatively charged groups). It will be shown below that the graphene derivative according to the instant invention is able to interact, e.g., with an orthopox virus. Thus, it shows virus-binding properties and can thus be used as anti-viral agent.

The modified graphene layer can shield a plurality of binding domains of the viruses due to its flexibility. Thus, upon binding of the modified graphene layer via the covalently bound polyglycerol compound, further sites of molecular interaction of the viruses are shielded. Taking into account multi-valence effects, such shielding of individual binding sites leads to a significant decrease in binding efficiency between the virus and potential binding sites on host cells.

In an embodiment, the polyglycerol compound has a dendritic backbone, in particular a hyperbranched backbone. Thus, in this embodiment, it can be denoted as dendritic polyglycerol (dPG) or hyperbranched polyglycerol (hPG). In case of sulfate groups as substituents, the polyglycerol can be denoted as dendritic polyglycerol sulfate (dPGS) or hyperbranched polyglycerol sulfate (hPGS). Dendritic polyglycerol sulfates (dPGS) and hyperbranched polyglycerol sulfates (hPGS) express little or no anticoagulant effect.

In an embodiment, the polyglycerol compound has a linear backbone. Thus, in this embodiment, it can be denoted as linear polyglycerol (IPG).

In an embodiment, the graphene derivative as described above is used in vitro for binding viruses. While almost any viruses can be bound by the graphene derivative, binding of orthopox virus particles such as variola virus, vaccinia virus (VACV), cowpox virus (CPXV) or monkeypox virus (MPXV) is particularly suited.

The variola virus caused about 300 million deaths alone in the 20^{th} century. Despite the eradication of the variola virus as a consequence of a global vaccination campaign by the World Health Organization (WHO), there is renewed interest in orthopoxvirus infections, partly due to the potential threat of variola virus as a biological weapon, but also because of zoonotic infections by related strains which pose a threat to public health. Zoonotic infections are caused, e.g., by VACV, CPXV or MPXV. Since all members of this genus demonstrate a significant genetic and morphological homology, antibody responses to these viruses show high cross-reactivity and cross-protectivity. In combination with good handling and a low security level (S2), vaccinia virus is a very well suited prototype for studies on all orthopox viruses.

The graphene derivative is, in an embodiment, dispersable in water or in aqueous solutions. In an embodiment, it is used in vitro for binding viruses. This means, it can extract viruses from aqueous solutions or suspensions, thus leading to agglutination of the viruses.

In an embodiment, the graphene derivative can also be used as superabsorber in masks (such as breathing masks). In an embodiment, the graphene derivative can be used as filter material for decontaminating liquids such as water like, e.g., sewage water.

It is possible to eliminate bound or agglutinated viruses by a photo thermal treatment with infrared (IR) radiation. This is due to the fact that graphene (also in its substituted form as instantly claimed) has a very good IR absorption efficiency. Thus, irradiation with IR radiation leads to a specific increase in temperature of the graphene derivative so that the viruses bound to the graphene derivative are efficiently thermally inactivated.

The instantly claimed graphene derivative is very well suited as anti-viral medicament as outlined above. Therefore, the invention relates, in an aspect, also to the use of this graphene derivative as a drug.

In an embodiment, the drug is a drug against viral diseases. Thus, the graphene derivative is particularly used as an anti-viral drug. It might be used in form of a virus absorber, e.g., to bind a virus, such as norovirus, directly in the gastrointestinal tract. It might also be used in a semisolid formulation, such as gel or salve, for virus inhibition, e.g., for inhibiting herpes or pox viruses.

An anti-viral drug comprising a graphene derivative according to the above-given explanations is also claimed.

It is also possible to use the graphene derivative as diagnostic tool for the detection of viruses, in particular of specific viruses. However, since the graphene derivative binds to many viruses, its specificity has to be carefully adjusted to achieve the desired specificity. This can be done, e.g., by adjusting the degree of substitution, as already outlined above. In addition, different substitutes of the covalently bound polyglycerol compound allow binding of different viruses.

The graphene derivative according to the preceding explanations can also be used as filtration system in vitro or in vivo. Thereby, the graphene derivative is in particular suited to be used as filtration material in a filter system.

The graphene derivative according to the preceding explanations can also be used as medical device in vitro or in vivo.

The graphene derivatives as described above can be manufactured is in general by two main manufacturing routes. The first route can be denoted as "graft from" manufacturing route and will be explained in the following.

In a first step, at least one graphene layer substituted by hydroxyl groups is provided. As outlined above, a suited possibility for providing such a substituted graphene layer can be the oxidation of graphite to graphite oxide (GO) and the subsequent treatment of the graphite oxide to obtain thermally reduced graphite oxide (TRGO). Both GO and TRGO can be used as graphene starting material within the teaching of the instant invention.

In the next step, the substituted graphene layer is mixed with glycidol. This step can be supported by a sonication, e.g., for a time period of 30 minutes to 4 hours, in particular 1 hour to 3 hours, in particular around 2 hours.

Then, the glycidol is reacted on the graphene layer to form polyglycerol that is covalently bound to the graphene layer. This is achieved by a ring opening polymerization of glycidol in a bulk reaction. Suited reaction conditions are a temperature between 100 and 200 °C, in particular between 110 and 190 °C, in particular between 120 and 180 °C, in particular between 130 and 170 °C, in particular between 140 and 160 °C, in particular between 145 and 150 °C. Suited reaction times are at least 5 hours, in particular between 5 and 30 hours, in particular between 10 and 24 hours, in particular between 15 and 20 hours. It is possible to stop the reaction by performing a quenching with an alcohol, such as methanol. For purification, a centrifugation to remove unreacted glycidol and free polyglycerol is suited. For further purification, it is possible to dialyze the precipitates against a suited solution, such as distilled water or an aqueous buffer solution.

In a next step, the covalently bound polyglycerol is modified with a negatively charged group so as to obtain a substituted polyglycerol. This is achieved by adding a compound to the graphene layer bearing the covalently bound polyglycerol, the compound being able to transfer a sulfate group onto the polyglycerol. Thus, the compound can be denoted as sulfation reagent. In an aspect of the invention, the compound is not necessarily suited to transfer only a sulfate group but rather to transfer any negatively charged group onto the polyglycerol. To give an example, sulfur trioxide pyridine is well suited to transfer a negatively charged group in form of sulfate onto the polyglycerol so as to produce a polyglycerol sulfate covalently bound to the graphene layer.

Suited conditions for this reaction are dispersing the polyglycerol-modified graphene layer in a hydrophobic solvent such as dimethyl formamide (DMF) and performing the reaction at elevated temperatures of between 40 and 100 °C, in particular between 50 and 90 °C, in particular between 60 and 80 °C, in particular between 65 and 70 °C. It is possible to work under inert atmosphere, e.g., by using an inert gas. Suited reaction times are at least 5 hours, in particular between 5 and 30 hours, in particular between 10 and 24 hours, in particular between 15 and 20 hours. It is possible to stop the reaction by quenching it with distilled water. In addition, it is possible to then adjust the pH to a basic value, such as between pH 7.5 and 12, in particular between 8 and 11, in particular between 9 and 10. It is possible to centrifuge the obtained reaction product and to dialyze it afterwards against a suited solvent, such as distilled water or an aqueous buffer solution.

The second manufacturing route can be denoted as "graft to" manufacturing route and will be explained in the following.

In a first step, at least one graphene layer substituted by functional groups such as hydroxyl groups, epoxy groups or carboxyl groups, is provided. As outlined above, a suited possibility for providing such as substituted graphene layer can be the oxidation of graphite to graphite oxide and the subsequent treatment of the graphite oxide to obtain thermally reduced graphite oxide (TRGO) if hydroxyl groups are to be chosen as functional groups.

In a second step, the substituted graphene layer is mixed with a grafting compound such as polyglycerol azide which is to be grafted to the substituted graphene layer. This leads to a reaction between the grafting compound and the functional groups of the substituted graphene layer, e.g., between an azide group of the polyglycerol azide and the graphene layer via a nitrene addition. In this example, an aziridine compound is formed and nitrogen is released. A suited possibility to achieve this reaction is to disperse hydroxide-substituted graphene in n-methyl-2-pyrrolidone under an inert atmosphere. Ultrasound can be applied to achieve proper dispersion. Then, the grafting compound, e.g., the polyglycerol azide, is added, and the reaction mixture is heated up to temperature of between 100 and 200 °C, in particular between 110 and 190 °C, in particular between 120 and 180 °C, in particular between 130 and 170 °C, in particular between 140 and 160 °C, in particular between 145 and 150 °C. Suited reaction times are at least 5 hours, in particular between 5 and 80 hours, in particular between 10 and 72 hours, in particular between 15 and 48 hours, in particular between 24 and 36 hours. It is possible to stop the reaction by quenching it with distilled water. It is possible to centrifuge the obtained reaction product and to dialyze it afterwards against a suited solvent, such as distilled water or an aqueous buffer solution.

In a third step, the covalently bound polyglycerol is modified with a negatively charged group so as to obtain a substituted polyglycerol. This can be done as described above with respect to the "graft from" manufacturing process.

In an embodiment, the compound being able to transfer a negatively charged group onto the polyglycerol compound is a sulfation reagent, such as sulfur trioxide pyridine. Then, the negatively charged group is a sulfate group.

All embodiments explained in connection to the claimed graphene derivative can also be applied to the described medicament, the described uses and the described manufacturing processes, and vice versa.

Further aspects and details of the invention will be explained with respect to figures and exemplary embodiments. In the Figures:
- FIG 1A: shows a schematic depiction of the molecular structure of graphite;
- FIG 1B: shows a schematic depiction of the molecular structure of graphite oxide;
- FIG 1C: shows a schematic depiction of the molecular structure of thermally reduced graphite oxide;
- FIG 2A: shows an embodiment of a "graft from" manufacturing process of a graphene derivative;
- FIG 2B: shows an embodiment of a "graft to" manufacturing process of a graphene derivative;
- FIG 3: shows a schematic depiction of the interaction between a graphene derivative and virus particles;
- FIG 4: shows the chemical structure of the polyglycerol backbone of an exemplary branched polyglycerol compound that can be covalently bound to thermally reduced graphite oxide;
- FIG 5A: shows a schematic depiction of TRGO;
- FIG 5B: shows a schematic depiction of dPG-derivatized TRGO;
- FIG 5C: shows a schematic depiction of dPGS-derivatized TRGO;
- FIG 5D: shows a schematic depiction of a virus bound to dPGS-derivatized TRGO;
- FIG 5E: shows the results of a thermogravimetric analysis of TRGO and two dPGS-derivatized TRGOs
- FIG 6A: shows a binding of orthopoxvirus strains to immobilized dPGS in comparison to dPG;
- FIG 6B: shows SPR inhibition experiments indicative of the inhibition of pox viral surface protein A27 by different substances;
- FIG 6C: shows the virus inhibition potential of dPGS in comparison to heparin;
- FIG 7A: shows a first graph on the inhibition of orthopoxvirus binding to specific A27 antibodies by different TRGO-dPGS derivatives;
- FIG 7B: shows a second graph on the inhibition of orthopoxvirus strains binding to specific A27 antibodies by a TRGO-dPGS derivative;
- FIG 8A: shows a first graph on the inhibition of virus infection by TRGO-dPG_{0.8}S_{0.5} in Hep2 cells;
- FIG 8B: shows a second graph on the inhibition of virus infection by TRGO-dPG_{0.8}S_{0.5} in Hep2 cells;
- FIG 9A: shows a first graph on the inhibition of virus infection by TRGO-dPG_{0.8}S_{0.5} in Hep2 cells;
- FIG 9B: shows a second graph on the inhibition of virus infection by TRGO-dPG_{0.8}S_{0.5} in Hep2 cells;
- FIG 9C: shows a third graph on the inhibition of virus infection by TRGO-dPG_{0.8}S_{0.5} in Hep2 cells;
- FIG 10: shows the results of an infrared irradiation of an exemplary embodiment;
- FIG 11: shows the results of an orthopoxvirus growth test bound to a TRGO-dPGS derivative after infrared irradiation;
- FIG 12: shows a graph on the reduction of infection of herpes viruses by a TRGO-dPGS derivative; and
- FIG 13: shows the results of an orthopoxvirus growth test bound to a TRGO-IPGS derivative after infrared irradiation.

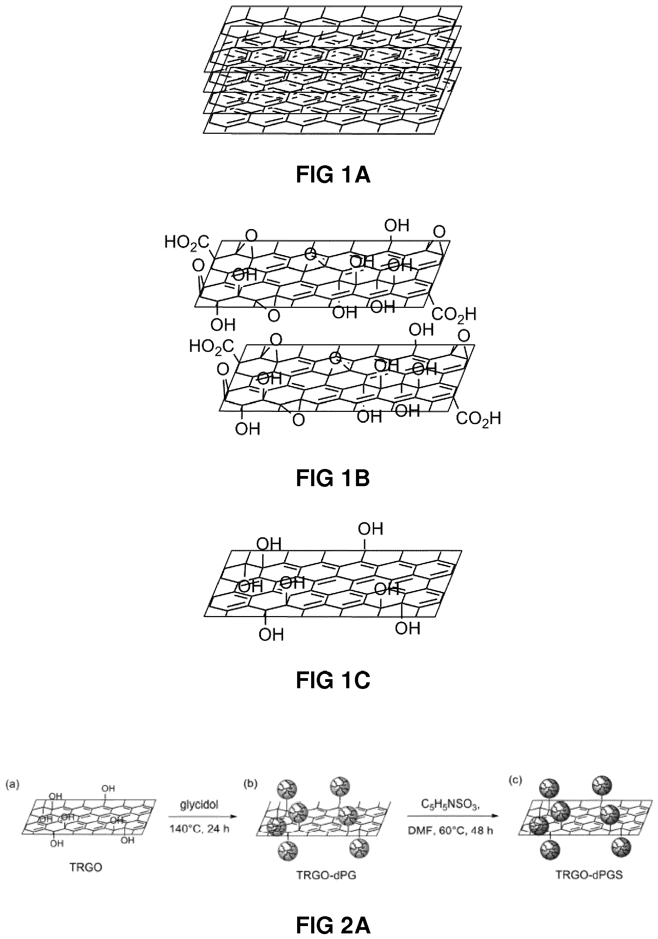

Figure 1A shows a schematic depiction of the molecular structure of graphite. It is composed of several layers of graphene stacked one above each other. Due to molecular layer-to-layer interactions, graphite is a very strong material.

By applying the Hummers-Offeman oxidation, graphite oxide (GO) is produced. This graphite oxide exhibits primarily epoxy and hydroxyl groups on the top and on the bottom of the individual layers of the graphite oxide. In addition, carboxyl groups are formed at the edges of the graphite oxide layers. This is shown in Figure 1B.

By an exfoliation, thermally reduced graphene oxide (TRGO) can be obtained. This thermally reduced graphene oxide is mainly composed of individual graphene layers substituted with hydroxyl groups. It is shown in Figure 1C.

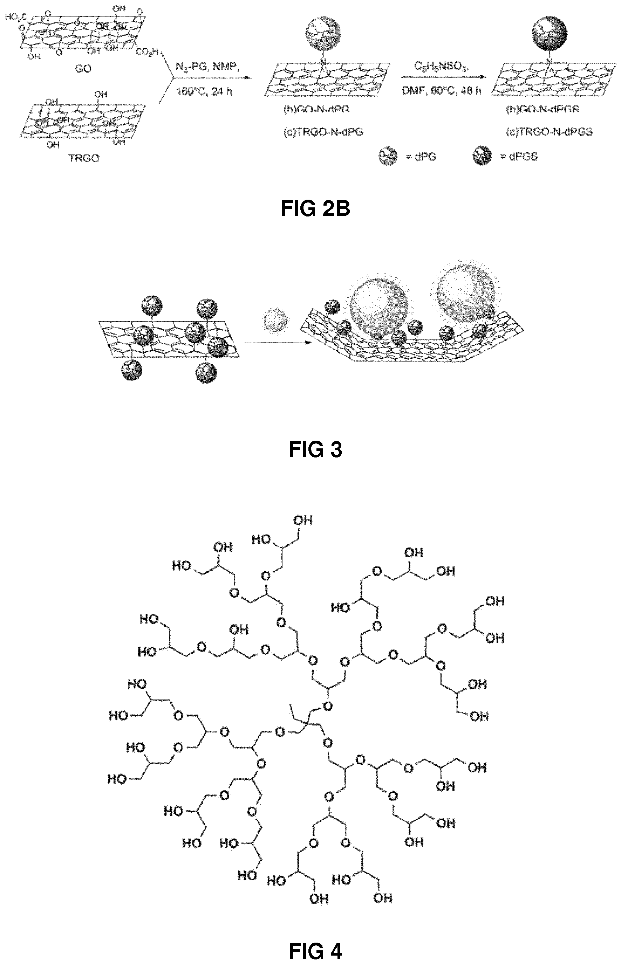

Figures 2A, 2B and 3 will now be explained in connection to exemplary embodiments of manufacturing graphene derivatives.

Air- and moisture-sensitive reactions were executed in flame-dried glassware under the usage of magnetic stirring, argon atmosphere and anhydrous solvents. All chemicals possessed reagent grade and were used without further purification. Dialyzes were carried on the one hand in benzoylated cellulose tubing (molecular weight cut off (MWCO) 2000 g mol⁻¹) and on the other hand in cellulose ester tubing (MWCO 20000 g mol⁻¹) changing the solvent at least 8 times over a period of 72 h. Centrifugation was carried out in a Rotina 380R. Raman spectra have been recorded on a Jobin-Yvon T64000 using a MBR-110 laser connected to a MBD-200 frequency doubler. XPS analysis were done by the Federal Institute for Materials Research and Testing (Berlin, Germany) on an ESCA spectrometer AXIS Ultra DLD. Thermogravimetric analysis have been recorded on STA PT 1600 and were evaluated with Linseis Data Acquisition software. The measurements were performed in aluminum oxide crucibles in a temperature range from 20 °C to 800 °C with a heating rate of 10 °C/min. ¹H-/¹³C-NMR C spectra have been recorded on a Jeol ECX 400 (400/ 101 MHz) or on a Bruker Biospin Avance 700 (700/ 176 MHz) spectrometer. In case of the ¹³C-NMR spectra a broadband proton decoupling was carried out. Multiplicity is labeled as s=singlet and m=multiplet. The chemical shifts δ for the proton as well as for the carbon NMR spectra were recorded in ppm and referenced to the deuterated solvent peak (CDCl₃: δ (¹H) = 7.26 ppm, δ (¹³C) = 77.16 ppm; CD₃OD: δ (¹H) = 3.31 ppm, δ (¹³C) = 49.00 ppm; D₂O: δ (¹H) = 4.79 ppm). IR measurements were recorded on a Nicolet Avatar 320 FT-IR operating with a DTGS detector in the range of 4000 to 650 cm⁻¹. Evaluation of the recorded data was done with EZ OMNIC E.S.P. software and the absorption intensities were categorized as b = broad, l = large, m = medium, s = small, vs = very small. Elemental analysis were carried out on a VARIO EL III instrument using sulfanilic acid as standard.

Virus interaction was determined by an ELISA-based competitive assay. For the assay, heat inactivated (56 °C for 45 minutes) vaccinia virus strain Western Reserve (VACV WR ATCC VR-1354) propagated on Hep2 cells (ATCC HB-8065) was used. Virus titers were determined on Vero E6 cells (ECACC 85020206) described in plaque forming units per ml virus containing suspension (pfu/ml). In short, Maxisorb plates in 96-well format were coated with an anti-A27 antibody (200 ng/well). Serial dilutions of the graphene or polymer derivatives (ranging from 31.25 µg/mL up to 2000 µg/ml) were pre-incubated with virus suspension (25,000 pfu/mL) for 30 min at 37 °C and 400 rpm before the mixture was added to the wells and incubated for 2 hours at 37 °C and 600 rpm. Virus samples were pre-incubated either with GO/TRGO-dPG-OH, -dPGS, or heparan sulfate (HS, sodium salt from bovine kidney (H7640)). Samples containing only virus or only buffer served as controls. For the analysis, biotinylated anti-A27 antibody competitively binding to the virus was added to the wells. After 1 hour at 37 °C and 300 rpm, streptavidin linked poly-horseradish-peroxidase (1:2500 in blocking buffer) was added and incubated for another 30 minutes at 300 rpm and 37 °C. For the detection, TMB (3,3',5,5'-Tetramethylbenzidin) substrate was added. The enzymatic reaction was stopped after 15 min by adding 0.25 M sulfuric acid. Optical density was measured using a TECAN-reader at 450 nm. The half-maximal inhibitory concentration (IC50) was determined by setting the only virus containing control to 100 % binding and the buffer treated control to 0% binding using the log(inhibitor) vs. normalized response - variable slope equation of the GraphPad Prism 5 software. Experiments were repeated at least 3 times with the exception of HS, and samples were tested at least in duplicates.

### Preparation of Graphite Oxide (2)

Graphite oxide **(2)** was synthesized by a modified Hummers and Offeman oxidation [5, 6, 7]. In short, Pristine graphite **(1)** (60.0 g) was dispersed in concentrated H₂SO₄ (95 %, 1.4 l) at room temperature followed by adding NaNO₃ (30.0 g). The dispersion was cooled down to 0 °C and KMnO₄ (180.0 g) was added in small portions for a period of 5 h under continuous stirring. After two days the oxidation was quenched in ice water (2 l) and treated with H₂O₂ (3%, 200.0 mL). The synthesized graphite oxide (2) was filtered off and washed several times before drying under reduced pressure at 40 °C for 4 days. The product (2) was pulverized by the usage of a CyroMill (60 µm mesh) and dried after for additional 4 days.

The analysis of this graphite oxide (2) led to the following results:
**FTIR:** *fi* = 3417 (b), 1714 (s), 1556 (m), 1454 (s), 1225-1134 (b), 1067 (b), 941 (vs) cm⁻¹.
***Elementary analysis:* C:** 56.96 %, **H:** 2.58 %, **O***: 40.46 %
   *calculated based on the CHN values
*XPS:* C 1s: 284.6 (C-C sp²), 286.8 (C-O-C, C-O-H), 288.5 (COO, COOH) 290.3 (A-A*-satellite), 292.8 (A-A*-satellite); O 1s: 531.5 (org. O), 532.6 (org. O), 533.5 (org. O), 534.9 (H₂O), 536.5 (A-A*-satellite).

### Preparation of Thermally Reduced Graphite Oxide (3)

Thermally reduced graphite oxide **(3)** (TRGO) was prepared according to a published procedure [6, 7]. In short, graphite oxide **(2)** was reduced thermally to obtain a better exfoliation and to increase the specific surface area [7]. The reduction was carried out in a tube furnace under a nitrogen atmosphere at 400 °C. On account of this method the TRGO **(3)** can directly be used for post- modification without further purification.

The analysis of this thermally reduced graphite oxide (3) led to the following results:
**FTIR:** *fi*= 3417 (b), 1714 (s), 1556 (m), 1454 (s), 1225-1134 (b), 1067 (b), 941 (vs) cm⁻¹.
***Elementary analysis:* C:** 80.88 %, **H:** 1.19 %, **O***: 17.93 %
   *calculated based on the CHN values

### Functionalization of Polyglycerol (4)

The o-mesylpolyglycerol **(5)** containing 5 % mesyl groups was synthesized first and then converted into polyglycerol azide **(6)** according to the published procedures [8].

The analysis of the o-mesylpolyglycerol **(5)** led to the following results:
***¹HNMR*** (700 MHz, D₂O) δ 3.28 (s, OMs-groups), 3.59 -4.05 (m, broad, PG);
***¹³C*** NMR [8, 14] (700 MHz, D₂O) δ 36,5 (Me), 60.8 (CH₂OH), 62.6 (CH₂OH), 68.8 (CHOH), 69.2 (CH2), 70.4 (CHOH), 70.7 (CH2), 70.9 (CH2), 72.2 (CH2), 77.9 (CH), 79.5 (CH) ppm.

The analysis of the polyglycerol azide **(6)** led to the following results:
**FTIR:** *fi* = 3361 (b), 2870 (I), 2099 (I), 1455 (s), 1267 (s), 1067 (b, vibration), 867 cm⁻¹ (s, C-H deformation vibration).
***¹H NMR*** (700 MHz, D₂O) δ 3.60 - 4.05 (m, broad, PG);
***¹³C NMR*** [8, 14] (700 MHz, D₂O) δ 53.1 (functionalized primary PG-groups), 60.8 (functionalized secondary PG- groups), 62.6 (CH₂OH), 68.9 (CHOH), 69.1 (CH2), 70.4 (CHOH), 70.7 (CH2), 70.9 (CH2), 72.2 (CH2), 77.9 (CH), 79.4 (CH) ppm.

### Preparation of TRGO-Polyglycerol derivatives (7,8) by "graft from"

The preparation of the TRGO-dPG **(7)** has been done based on the method for nanodiamonds published by Komatsu et al. [9, 10]: The thermally reduced graphite oxide **(3)** (613.0 mg) and the recent distilled glycidol (60.0 mL) were sonicated under argon atmosphere in an ultrasonic bath at 35°C for 2 h to obtain a stable dispersion. After ultrasonication the dispersion was continuous stirred at 140°C for 24 h and then cooled down to room temperature before quenching with methanol (100.0 mL). The black dispersion was centrifuged five times (6000 rpm, 15 min) to remove the unreacted glycidol and the free polyglycerol. The precipitates were collected and dialyzed for 3 days in distilled water to remove the remaining methanol. Finally a black solid **(7)** was obtained as product after drying in the lyophilizer.

The preparation of the TRGO-dPG **(8)** has been done in the same way as described above by using graphite oxide (50.0 mg) and recent distilled glycidol (6.0 ml).

The analysis of this TRGO-Polyglycerol derivative **(7)** led to the following results:
**FTIR:** *fi* = 3319 (b), 2867 (l), 1556 (m), 1454 (s), 1323 (s), 1241 (s), 1036 (b), 836 (vs) cm⁻¹.
***¹H NMR*** (700 MHz, D₂O) δ 3.58 - 4.04 (m, broad, PG);
***¹³C NMR*** (700 MHz, D₂O) δ 60.7 (CH₂OH), 62.6 (CH₂OH), 68.8 (CHOH), 69.2 (CH₂), 70.4 (CHOH), 70.7 (CH2), 70.9 (CH2), 72.1 (CH2), 77.9 (CH), 79.4 (CH) ppm.
***Elementary analysis:* C:** 54.43 %, **H**: 6.62 %, **O***: 38.95 %
   *calculated based on the CHN values
***XPS:*** C 1s: 284.6 (C-C sp²), 286.7 (C-O-C, C-O-H), 288.0 (C=O, C-O-C), 289.3 (COO, COOH); O 1s: 532.8 (org. O), 533.3 (org. O), 534.2 (H₂O).

The analysis of this TRGO-Polyglycerol derivative **(8)** led to the following results:
**FTIR:** *fi* = 3334 (b), 2869 (l), 1567 (m), 1453 (s), 1325 (s), 1240 (s), 1039 (b), 845 (vs) cm⁻¹.
***¹H NMR*** (700 MHz, D₂O) δ 3.57 - 4.04 (m, broad, PG);
***¹³C NMR*** (700 MHz, D₂O) δ 60.7 (CH₂OH), 62.6 (CH₂OH), 68.9 (CHOH), 69.2 (CH₂), 70.4 (CHOH), 70.7 (CH2), 70.9 (CH2), 72.1 (CH2), 77.9 (CH), 79.4 (CH) ppm.
***Elementary analysis:* C:** 50.31 %, **H**: 7.29 %, **O***: 42.29 %
   *calculated based on the CHN values

### Preparation of TRGO-Polyglycerol sulfate (9,10)

The TRGO-dPG **(7)** (320.0 mg) was dispersed in p.a. DMF (12 mL) using an ultrasonic bath at 35°C under inert conditions. After 30 min the stable dispersion was heated up to 60°C under continuous stirring before adding the sulfur trioxide pyridine complex drop wise over a period of 4 h. For this purpose the sulfur trioxide pyridine complex (825.0 mg, 5.2 mmol) was dissolved in p.a. DMF (8 mL) under argon atmosphere. The dispersion was kept at 60°C for 24 h before cooling down to room temperature. The reaction was quenched with distilled H₂O (20 ml) and the pH was adjusted to 8 by addition of NaOH. The black dispersion was centrifuged five times (6000 rpm, 15 min) before the precipitate was collected and dialyzed for 3 days in distilled water to remove the remaining DMF. A black fluffy solid was obtained as product TRGO-dPGS **(9)** after drying in the lyophilizer.

The analysis of this TRGO-Polyglycerol sulfate **(9)** led to the following results:
**FTIR:** *fi* = 3291 (b), 2867 (s), 1722 (vs), 1558 (m), 1116 (s), 1067 (b), 945 (vs) cm⁻¹.
***¹H NMR*** (700 MHz, D₂O) δ 3.85 - 4.35 (m, broad, PG).
***Elementary analysis:* C:** 47.09 %, **H**: 3.45, **N**: 0.52 %, **S**: 9.43 %, **O***: 32.62 %, **Na***: 6.78 % *calculated based on the CHNS values
***XPS*:** C 1s: 284.6 (C-C sp²), 286.3 (C-O-C, C-O-H), 287.6 (C=O, C-O-C), 288.9 (COO, COOH), 290.6 (A-A*-satellite); O 1s: 531.0 (org. O), 532.5 (org. O), 533.3 (org. O), 535.1 (H₂O), 536.5 (A-A*-satellite); S 2p_{3/2}: 168.9 (sulfate).

The TRGO-dPGS **(10)** was prepared in the same way as shown above but this time the following amounts have been used: TRGO-hPG **(8)** (300 mg), sulfur trioxide pyridine complex (1290 mg, 8.1 mmol) and p.a. DMF (20 mL) to obtain the higher sulfated TRGO-dPGS **(10).**

The analysis of this TRGO-Polyglycerol sulfate **(10)** led to the following results:
**FTIR:** *fi* = 3439 (b), 2876 (s), 1735 (vs), 1562 (m), 1108 (s), 1042 (b), 933 (vs) cm⁻¹.
***¹H NMR*** (700 MHz, D₂O) δ 3.80 - 4.65 (m, broad, PG).
***Elementary analysis:* C:** 27.75 %, **H:** 2.75, **N:** 0.42 %, **S:** 14.41 %, **O***: 44.33 %, **Na***: 10.33 % *calculated based on the CHNS values

### Preparation of GO-N-Polyglycerol (11) by "graft to"

One way for a "grafting to" method is a nitrene addition of azido compounds to a graphene layer [11-13] but it is also possible for GO and TRGO. In this "graft to" process GO **(2)** (100.0 mg) was well dispersed in n-methyl-2-pyrrolidone (20 mL) under argon atmosphere in 100 mL Schlenk flask by using a ultrasonic bath for 1 h. After this the solubilized hPG-azide **(6)** (400.0 mg in 5 mL NMP) was added to the black dispersion under continuous stirring and inert conditions. The reaction mixture was heated up to 160°C and stirred for 72 h before cooling down to room temperature and diluting with distilled water (30 mL). For purification the compound was centrifuged five times (6000 rpm, 15 min) before the precipitate was collected and dialyzed for 3 days in distilled water to remove the remaining n-methyl-2-pyrrolidone. After lyophilizing a black solid was obtained **(11).**

The analysis of this GO-N-Polyglycerol **(11)** led to the following results:
**FTIR:** *fi*= 3345 (b), 2869 (I), 2097 (vs), 1698 (vs), 1652 (vs), 1558 (m), 1434 (s), 1333 (s), 1251 (s), 1039 (b), 929 (vs), 849 (vs) cm⁻¹.
***¹H NMR*** (700 MHz, D₂O) δ 3.59 - 4.04 (m, broad, PG);
***¹³C NMR*** (700 MHz, D₂O) δ 60.8 (CH₂OH), 62.6 (CH₂OH), 68.9 (CHOH), 69.1 (CH₂), 70.4 (CHOH), 70.7 (CH2), 70.9 (CH2), 72.2 (CH2), 78.0 (CH), 79.5 (CH) ppm.
***Elementary analysis:* C**: 55.17 %, **H**: 6.25, **N**: 2.67 %, **O***: 35.98 %
   *calculated based on the CHN values
***XPS:*** C 1s: 284.6 (C-C sp²), 286.1 (C-O-C, C-O-H), 287.8 (C=O, C-O-C), 288.9 (COO, COOH), 293.5 (A-A*-satellite); O 1s: 531.1 (org. O), 532.4 (org. O), 533.8 (org. O); N 1s: 400.2 (-N-C=O, NC₃), 402.0 (NH₂/NH₃).

### Preparation of GO-N-Polyglycerol sulfate (12)

The GO-N-dPG **(11)** (20.0 mg) was dispersed in p.a. DMF (8 mL) using an ultrasonic bath at 35 °C under inert conditions. After 30 min the stable dispersion was heated up to 60 °C under continuous stirring before adding the sulfur trioxide pyridine complex drop wise over a period of 4 h. For this purpose the sulfur trioxide pyridine complex (214.9 mg, 1.4 mmol) was dissolved in p.a. DMF (2 mL) under argon atmosphere. The dispersion was kept at 60 °C for 24 h before cooling down to room temperature. The reaction was quenched with distilled H₂O (10 mL) and the pH was adjusted to 8 by addition of NaOH. The black dispersion was centrifuged five times (6000 rpm, 15 min) before the precipitate was collected and dialyzed for 3 days in distilled water to remove the remaining DMF. A black fluffy solid was obtained as product **(12)** after drying in the lyophilizer.

The analysis of this GO-N-Polyglycerol sulfate **(12)** led to the following results:
***¹H NMR*** (700 MHz, D₂O) δ 3.73 - 4.34 (m, broad, PG)
***Elementary analysis:* C:** 43.63 %, **H:** 1.16, **N:** 1.94 %, **S:** 9.54 %, **O***: 36.89 %, **Na***: 6.84 % *calculated based on the CHNS values
***XPS:*** C 1s: 284.6 (C-C sp²), 286.3 (C-O-C, C-O-H), 287.2 (C=O, C-O-C), 288.6 (COO, COOH); O 1s: 532.4 (org. O), 533.2 (org. O); S 2p_{3/2}: 169.8 (sulfate).

### Preparation of TRGO-N-Polyglycerol (13) by "graft to"

In this "graft to" process, TRGO **(3)** (125.0 mg) was well dispersed in n-methyl-2-pyrrolidone (20 mL) under argon atmosphere in 100 ml Schlenk flask by using a ultrasonic bath for 1 h. After this the solubilized hPG-azide **(6)** (500.0 mg in 5 mL NMP) was added to the black dispersion under continuous stirring and inert conditions. The reaction mixture was heated up to 160 °C and stirred for 72 h before cooling down to room temperature and diluting with distilled water (30 mL). For purification the compound was centrifuged five times (6000 rpm, 15 min) before the precipitate was collected and dialyzed for 3 days in distilled water to remove the remaining n-methyl-2-pyrrolidone. After lyophilizing a black solid **(13)** was obtained.

The analysis of this TRGO-N-Polyglycerol **(13)** led to the following results:
**FTIR:** *fi*= 3089 (b), 2868 (b), 1706 (vs), 1548 (m), 1215 (m), 1064 (b), 963 (vs) cm⁻¹.
***¹H NMR*** (700 MHz, D₂O) δ 3.66 - 4.05 (m, broad, PG);
***¹³C NMR*** (700 MHz, D₂O) δ 54.9 (functionalized primary PG-groups), 60.8 functionalized secondary PG- groups), 62.6 (CH₂OH), 68.9 (CHOH), 69.2 (CH2), 70.4 (CHOH), 70.7 (CH2), 70.9 (CH₂), 72.1 (CH₂), 78.1 (CH), 79.4 (CH) ppm.
***Elementary analysis:* C:** 66.95 %, **H:** 3.26, **N:** 1.02 %, **O***: 28.77 %
   *calculated based on the CHN values
***XPS***: C 1s: 284.6 (C-C sp²), 286.4 (C-O-C, C-O-H), 288.1 (C=O, C-O-C), 289.4 (COO, COOH), 290.9 (A-A*-satellite); O 1s: 530.0 (MeₓO_{y}), 530.9 (org. O), 532.9 (org. O), 534.2 (H₂O).

### Preparation of TRGO-N-Polyglycerol sulfate (14)

The TRGO-N-dPG **(13)** (50.0 mg) was dispersed in p.a. DMF (11 mL) using an ultrasonic bath at 35 °C under inert conditions. After 30 min the stable dispersion was heated up to 60 °C under continuous stirring before adding the sulfur trioxide pyridine complex drop wise over a period of 4 h. For this purpose the sulfur trioxide pyridine complex (537.2 mg, 3.8 mmol) was dissolved in p.a. DMF (4 mL) under argon atmosphere. The dispersion was kept at 60 °C for 24 h before cooling down to room temperature. The reaction was quenched with distilled H₂O (20 mL) and the pH was adjusted to pH 8 by addition of NaOH. The black dispersion was centrifuged five times (6000 rpm, 15 min) before the precipitate was collected and dialyzed for 3 days in distilled water to remove the remaining DMF. A black fluffy solid was obtained as product **(14)** after drying in the lyophilizer.

The analysis of this TRGO-N-Polyglycerol sulfate **(14)** led to the following results:
**FTIR:** *fi*= 3390 (s), 2857 (vs), 1729 (vs), 1555 (m), 1454 (s), 1329 (s), 1209 (s), 1116 (b), 941 (b) cm⁻¹.
***¹H NMR*** (700 MHz, D₂O) δ 3.7 - 4.34 (m, broad, PG)
***Elementary analysis:* C:** 54.03 %, **H:** 2.01, **N:** 1.75 %, **S:** 6.22 %, **O***: 31.53 %, **Na***: 4.46 % *calculated based on the CHNS values
***XPS***: C 1s: 284.6 (C-C sp²), 286.4 (C-O-C, C-O-H), 288.9 (COO, COOH); O 1s: 530.6 (org. O), 532.0 (org. O), 533.2 (org. O), 534.2 (H₂O); S 2p_{3/2}: 169.0 (sulfate).

The results of the elementary analyses of some of the produced compounds and intermediary compounds are also summarized the following tables 1 and 2.

**Table 1: C-H-N Analysis of GO and TRGO as well as the "grafted to" GO-N-hPG (11) and the TRGO-N-hPG (13)**

| **Sample** | **C [%]** | **H [%**] | **N [%**] | **O* [%]** |
|---|---|---|---|---|
| **GO (2)** | 56.79 | 2.49 | 0.00 | 40.72 |
| **GO-N-hPG (11)** | 55.17 | 6.25 | 2.67 | 35.98 |
| **TRGO 400 (3)** | 80.89 | 1.19 | 0.00 | 17.92 |
| **TRGO-N-hPG (13)** | 66.95 | 3.26 | 1.02 | 28.77 |

| | | | | |
|---|---|---|---|---|
| *Approximation based on the C-H-N values | | | | |

**Table 2: C-H-N Analysis of the "grafted from" TRGO-hPGS (9) and TRGO-hPGS (10) and the "grafted to" GO-N-hPGS (12)**

| **Sample** | **C [%]** | **H [%]** | **N [%]** | **S [%]** |
|---|---|---|---|---|
| **TRGO-hPGS (9)** | 47.09 | 3.45 | 0.52 | 9.43 |
| **TRGO-hPGS (10)** | 27.75 | 2.75 | 0.42 | 14.41 |
| **GO-N-hPGS (12)** | 43.63 | 1.16 | 1.94 | 9.54 |

| | | | | |
|---|---|---|---|---|
| *Approximation based on the C-H-N-S values | | | | |

Figure 4 is a schematic depiction of the polyglycerol backbone of an exemplary hyperbranched polyglycerol (hPG). Such dPG can be used as starting point for preparing an azide-substituted PG that in turn can be covalently bound to graphene or GO or TRGO, respectively. The dPG as depicted in Figure 4 has a number average molecular mass (Mₙ) of approximately 5000 g·mol⁻¹. It was prepared by a one-step anionic ring-opening polymerization, as previously described [14, 15].

Aspects of isolated dendritic polyglycerol sulfates have already been described previously [17, 18].

Exemplary embodiments of graphene derivatives according to the invention were tested with respect to their multivalent interaction with orthopoxvirus particles and for inhibition of orthopoxvirus infection. Orthopoxviruses are double-stranded (ds) DNA viruses with a size of 300 x 400 nm, which replicate in the cytoplasm of infected host cells.

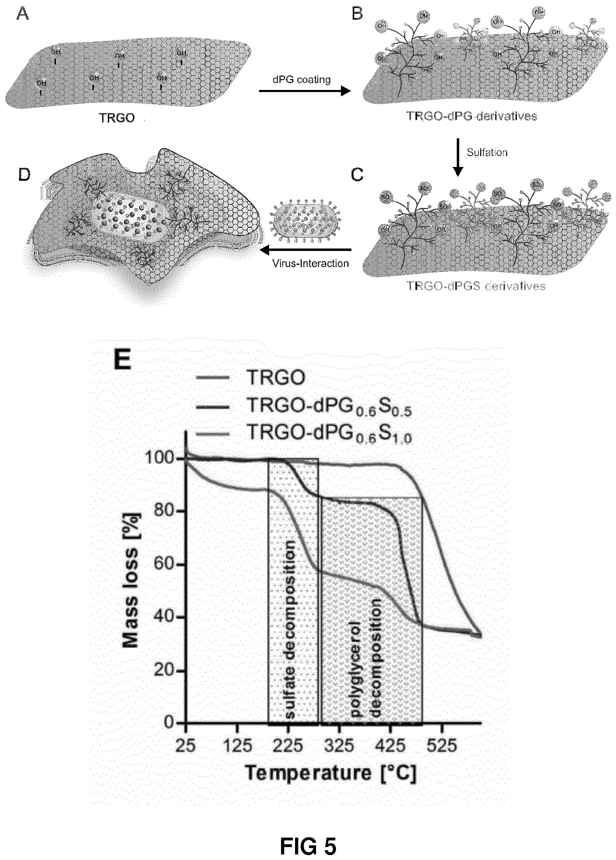

Figures 5A to 5D show a schematic representation TRGO and derivatized TRGO in different stages. Figure 5A schematically depicts thermally reduced graphite oxide (TRGO). Figure 5B schematically depicts polyglycerol functionalized TRGO derivatives (TRGO-dPG) produced by a "grafting from" method. Figure 5C schematically depicts polysulfated dPG-functionalized TRGO derivatives (TRGO-dPGS). Figure 5D schematically depicts an interaction of an orthopoxvirus particle with a TRGO-dPGS sheet.

Figure 5E depicts the results of a thermogravimetric analysis (TGA), namely TGA curves recorded from 25 °C to 600 °C for the two lower functionalized derivatives TRGO-dPG_{0.6}S_{0.5} and TRGO-dPG_{0.6}S_{1.0}. From these curves, it can be seen that a mass loss occurs already at low temperatures in case of TRGO-dPG_{0.6}S_{1.0} (mainly due to the loss of sulfate groups at temperatures below approximately 300 °C), whereas non-derivatized TRGO loses part of its mass only at very high temperatures (above 425 °C).

As exemplary embodiments, multivalent 2D architectures with varying degrees of sulfation and grafting densities were synthesized to elucidate the influence on virus interaction and binding efficiency. In addition to the experimental data presented above, additional data was obtained that will be explained in the following.

### Functionalization of multivalent 2D architectures

Upon preparation of dendritic polyglycerol-coated TRGO (TRGO-dPG) by a "grafting from" approach, data from FTIR spectroscopy, X-ray photoelectron spectroscopy (XPS), and elementary analysis were recorded to characterize the respective products (see also above). During the thermal reduction of the GO to TRGO, many functional groups were removed from the surface. This process can be observed in the FTIR spectra. In the case of TRGO the bands for the C=O stretching vibration at around 1714 cm⁻¹ almost vanished and the C-O stretching vibration in the region 1225 to 1134 cm⁻¹ massively decreased in intensity. By comparing the FTIR spectra of TRGO and TRGO-dPG, an increase was detected for the broad band of the O- H stretching vibration and for the C-O stretching vibration peak at 1241 cm⁻¹ as a result of the grafted polyglycerol on the carbon sheets. Furthermore, the large peak for the C-H stretching vibration of the polyglycerol appeared in the region 2900 to 2850 cm⁻¹, while the significant band for the C=C stretching vibration at 1556 cm⁻¹ of the carbon layer decreased. The same trend could be seen in XPS spectra for the TRGO-dPG by inspecting the intensities of the binding energies for C-C sp² at 284.6 eV and for C-O-C at 286.7 eV.

An elementary analysis allowed to reconstruct every single step of the functionalization process of the TRGO-dPG. From the pristine graphite via Hummers-Offeman oxidation to the GO, the carbon content dramatically decreased because of the insertion of the functionalities (epoxy, hydroxyl, and carboxylic groups). During thermal reduction at 400 °C, the amount of functional groups decreased and the carbon content rose simultaneously. The ring-opening polymerization of glycidol directly led to a massive increase of the hydroxyl groups, which could also be observed in the FTIR spectra.

The recorded data indicates that the "grafting from" approach was successful and different TRGO-dPG derivatives were obtained. The lower functionalized TRGO-dPG_{0.6} (dPG_{0.6} = dPG content of 60%) as well as the higher functionalized TRGO-dPG_{0.8} (dPG_{0.8} = dPG content of 80%) were post-modified by sulfation (Table 3) and tested in biological virus-binding studies.

The final post-modification was carried out for both TRGO-dPG derivatives to obtain four different multivalent 2D architectures which mimic the heparan sulfate-rich cell surface and are able to bind orthopoxvirus particles (cf. Figures 5A to 5D). For this step a partial conversion of the existing hydroxyl groups from the covalently attached dendritic polyglycerol on the honeycomb layers into sulfates was performed. By using a sulfur trioxide pyridine complex, a specific targeted amount of sulfates could be generated that allowed the multivalent systems to interact with different architectures through a charge interaction. The degree of sulfation was varied to study the resulting impact on the binding efficiency of the functionalized carbon material in more detail. From the elementary analysis results and by comparing the mass loss of the synthesized derivatives due to thermal decomposition (cf. Figure 5E) in TGA, it is obvious that the amount of sulfates for the TRGO-dPG_{0.6}S_{1.0} (S_{1.0} = 100% conversion of the hydroxyl groups into sulfates) is much higher than for the TRGO-dPG_{0.6}S_{0.5} (S₀.₅ = 50% conversion of the hydroxyl groups into sulfates).

The first mass loss occurred at a temperature ranging between about 200 °C and 280 °C and represents the decomposition of the sulfate groups, while the mass loss at a temperature ranging between 280 °C and 500 °C was due to the decomposed dendritic polyglycerol. Based on these observations, the amount of sulfate groups for TRGO-dPG_{0.6}S_{1.0} was approximately two-fold higher than for the TRGO-dPG_{0.6}S_{0.5} (cf. Table 3). For the higher functionalized pair the TGA showed that the amount of sulfate groups for TRGO-dPG_{0.8}S_{0.5} was approximately ten-fold higher than for TRGO-dPG_{0.8}S_{0.05}. In the following experiments, these four synthesized TRGO-dPGS derivatives were further characterized concerning their potential to act as multivalent inhibitors of poxvirus infections.

### Interaction of carbon-based dPGS functionalized architectures with orthopoxvirus particles

The experiments described in the following were performed in order to show that the dPGS functionalized surface interacts with orthopoxvirus particles and enables specific binding to the heparin-binding poxviral surface protein A27.

The A27 protein expressed on the cell surface of mature virions (MV) of orthopoxviruses exhibits a heparin binding domain, enabling the binding to heparin as well as heparan sulfate. Various studies describe the inhibitory potential regarding viral cell attachment of soluble heparin due to its competitive binding to viral A27.

Biotinylated dPGS (95% sulfated) as well as biotinylated dPG (unsulfated control) were immobilized on microtiter plates. Subsequently, binding of three different native poxvirus strains (VACV Western Reserve, VACV WR; VACV International Health Department, VACV IHD-W; Cowpox Virus Brighton Red, CPXV BR) was tested in an enzyme linked lectin assay (ELLA)-like format. Bound virus was detected via anti-A27-specific antibodies. All virus strains showed specific binding to immobilized dPGS while only weak, unspecific binding to the bare plate and immobilized dPG was observed at very high virus concentrations.

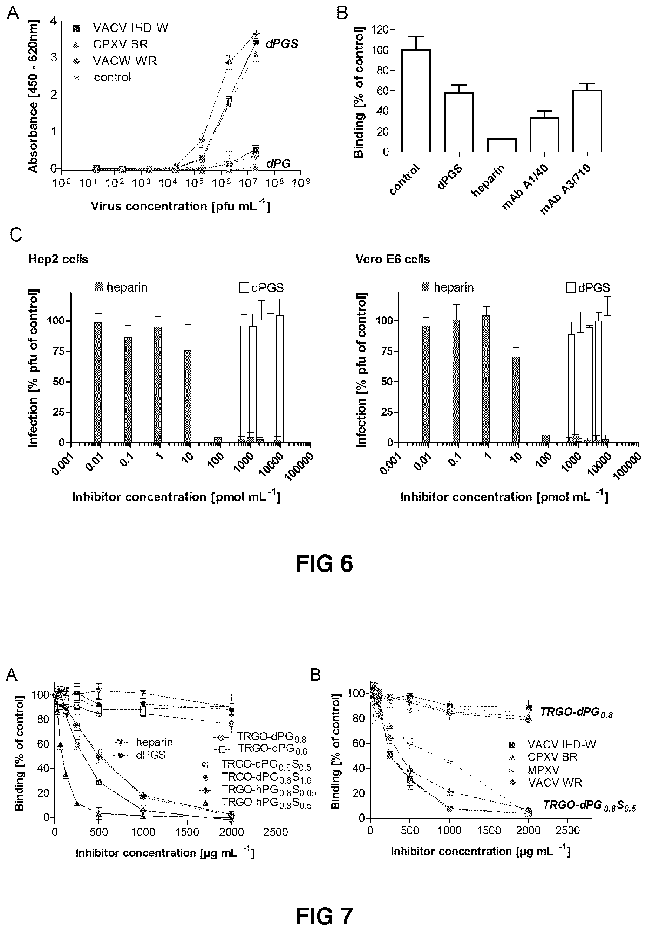

The results are shown in Figures 6A to 6D showing the binding characteristics of dPGS to orthopoxvirus particles and the orthopoxvirus protein A27. Figure 6A shows the binding of orthopoxvirus strains to immobilized dPGS in comparison to dPG analyzed by an enzyme linked lectin assay (ELLA)-like format. Dilutions of three native orthopoxvirus strains (Vaccinia Virus Western Reserve, VACV WR: n=3, ±SD; Vaccinia Virus International Health Department, VACV IHD-W: n=2, ±SD; Cowpox Virus Brighton Red, CPXV BR: n=2, ±SD) were added to plates coated with either dPGS or dPG and binding was determined using anti-A27 antibodies. As a control non-specific binding of VACV IHD-W to the bare plate was tested (n=2, ±SD).

These results show that a dPGS-functionalized surface is able to mimic the heparan sulfate-containing cell surface and enables the binding of different orthopoxviruses. To further confirm these results, SPR measurements were performed. It could be shown that the poxviral surface protein A27 binds to heparan sulfate as well as to dPGS. However, the interaction of A27 with immobilized dPGS seems to be characterized by a faster association rate compared to heparan sulfate. As it is known that charge interactions, like those between the positively charged heparin binding domain of A27 and the negatively charged heparan sulfate, largely contribute to an increase in association rates, the differences in the binding characteristics most likely reflect the higher proportion of sulfate residues found in dPGS compared to heparan sulfate. The results show that both the binding of A27 to dPGS as well as to heparan sulfate rely mainly on electrostatic interaction, which underlines the ability of bound dPGS to mimic cell surface-bound heparan sulfate.

To evaluate the possible inhibition potential of free dPGS towards A27 binding to heparan sulfate, SPR inhibition experiments were performed. Heparin was used as control, which is already known as virus inhibitor as well as two anti-A27 mAbs (A1/40 and A3/710) that bind adjacent to the heparin binding domain of A27. The pre-incubation of A27 protein with free dPGS, heparin, as well as A27-specific antibodies resulted in significantly reduced binding to immobilized heparan sulfate.

This is shown in Figure 6B depicting SPR inhibition experiments showing inhibition of poxviral surface protein A27 binding to heparan sulfate by dPGS in comparison to heparin. A27 was incubated with unbound heparin or dPGS and as control with A27-specific antibodies mAb A1/40 or mAb A3/710 prior to injection to a heparan sulfate-coated chip mimicking the cell surface. Non-treated A27 served as control and was set to 100% (n=2, ±SD).

The most potent inhibition was achieved by unbound heparin which reduced A27 binding by more than 90%. Conversely, free dPGS, and the A27-specific antibodies showed a milder inhibition between 85% and 60%, indicating an incomplete shielding of heparin binding sites.

Furthermore, a cell based infection assay - a plaque reduction neutralization test (PRNT) - was performed to confirm the inhibition potential of dPGS in comparison to heparin in a more natural setting in vitro.

The results are shown in Figure 6C depicting the evaluation of the virus inhibition potential of dPGS in comparison to heparin for Hep2 cells (left panel) and Vero E6 cells (right panel). The PRNT was performed using Vaccinia Virus International Health Department (VACV IHD-W: n=3, ±SD). Virus was pre-incubated with dPGS or heparin in serial dilutions for 30 minutes and then added to the cells and incubated for 1 h. Plaques were counted 4 days after infection. Concentrations are given in pmol mL⁻¹.

The PRNT results clearly show that free dPGS cannot inhibit viral infections in the two tested cell lines, whereas the natural occurring heparin shows good viral inhibition of poxvirus infection in both cell lines.

The strong inhibitory effect of heparin could be attributed to the high sulfate content and its linear structure which provides a higher contact area and by this, an increased multimeric binding compared to spherical, free dPGS. Spherical dPGS seems not to be able to completely shield the A27 protein, resulting in incomplete coverage of heparin binding domains. However, A27 displays a faster association to immobilized dPGS than it does to immobilized heparan sulfate.

Therefore, the inventors propose that dPGS is able to bind and shield the A27 protein by multivalent interactions if its contact area is increased by coating it to a surface. This observation indicates the major need for a flexible and highly functionalized system like the above introduced 2D polymer architectures. For this purpose, further experiments were performed using TRGO derivatives functionalized with dPGS.

**Table 3. The degree of functionalization (DF) and sulfation (DS) for the four designed TRGO-dPGS are approximated calculation results, which are based on the data from elementary analysis results and the TGA results (Figure 5E). The half maximal inhibitory concentration (IC50) was determined using the log (inhibitor) vs. normalized response - variable slope equation of the GraphPad Prism 5 software.**

| **Derivatives** | **IC50 Binding [µg mL⁻¹]** (95% confidence interval) | **DF [%]** | **DS [%]** |
|---|---|---|---|
| TRGO-dPG_{0.6}S_{0.5} | 470 (424-520) | 63 | 52 |
| TRGO-dPG_{0.6}S_{1.0} | 301 (282-322) | 63 | 100 |
| TRGO-dPG_{0.8}S_{0.05} | 470 (436-505) | 82 | 5 |
| TRGO-dPG_{0.8}S_{0.5} | 84 (79-89) | 75 | 49 |

An ELISA based assay was used to assess the inhibitory potency of our newly synthesized TRGO-dPGS derivatives. This assay offered the possibility to screen the different derivatives under a decreased containment level in a more efficient, economic way. To this aim, four derivatives were analyzed that differed in their grade of functionalization and sulfation (Table 3). Free dPGS, heparin, TRGO-dPG_{0.8} and TRGO-dPG_{0.6} were used as controls. Binding of the derivatives to the virus was expected to decrease the signal due to the shielding of the virus by the examined compounds and the concomitant inhibition of the antibody binding. Strong inhibition of antibody binding was observed for all four TRGO-dPGS derivatives.

The results are shown in Figure 7A depicting the inhibition of orthopoxvirus binding to specific A27 antibodies by different TRGO-dPGS derivatives using inactivated VACV WR tested in an ELISA-based format. Data points represent the mean of at least three independent experiments (n≥3) with ±SD.

The TRGO-dPG_{0.8}S_{0.5} showed the strongest inhibitory effect with an IC₅₀ of 84 µg mL⁻¹, which is about one fifth of the IC₅₀ of TRGO-dPG_{0.8}S_{0.05} of 470 µg mL⁻¹. The difference can be attributed to the grade of sulfation since both substances are products of the same precursor and only differ in their number of sulfate residues, with TRGO-dPG_{0.8}S_{0.05} expressing fewer sulfate moieties (cf. Table 3).

The same trend can be observed for the lower functionalized substance pair TRGO-dPG_{0.6}S_{1.0} and TRGO-dPG_{0.6}S_{0.5} by comparing the IC₅₀ values which correlate with the grade of sulfation (301 µg mL⁻¹ versus 470 µg mL⁻¹), respectively. Interestingly, TRGO-dPG_{0.8}S_{8.5}, which has a lower overall sulfate content than TRGO-dPG_{0.6}S_{1.0} and even the same sulfate content as TRGO-dPG_{0.6}S_{0.5} but a higher functionalization, showed a much stronger binding inhibition, highlighting the importance of high dPG functionalization and medium sulfation.

The positive influence of a high dPG functionalization was further stressed, since the higher functionalized TRGO-dPG_{0.8}S_{0.05} with the lowest sulfate content of all derivatives showed at least the same inhibition as the lower functionalized but higher sulfated TRGO-dPG_{0.6}S_{0.5}. A possible explanation of this behavior is steric crowding upon higher sulfation or most likely the size-dependent effect of a higher multivalent surface due to the higher dPG content.

In contrast, heparin, which can be used as viral entry inhibitor, and its synthetic analog dPGS did not show any significant inhibition of antibody binding in the ELISA based assay. This implies that the multimeric presentation of the A27 protein allows antibody-binding even in the presence of heparin or dPGS. Additionally, hydroxy terminated TRGO-dPG derivatives did not show any remarkable binding competition (cf. Figure 7A). Moreover, the binding inhibition of native poxvirus strains (including VACV WR, VACV IHD-W, CPXV BR, and MPXV) was successfully achieved as exemplarily tested for the highly sulfated graphene derivate TRGO-dPG_{0.6}S_{1.0}.

These results are shown in Figure 7B depicting the inhibition of native orthopoxvirus strains (VACV WR: n=3, ±SD; VACV IHD-W: n=2, ±SD; CPXV BR: n=2, ±SD; Monkeypox Virus, MPXV: n=2, ±SD) to specific A27 antibodies by TRGO-dPG_{0.6}S_{1.0}. TRGO-dPG_{0.6} served as control.

The data of the virus interaction assay provides strong evidence for the fact that the examined 2D polysulfates bind to orthopoxviruses and efficiently shield these particles. Since anti-A27 antibodies were used in the assay, A27 in the virus membrane is most likely involved in this binding, or at least the accessibility to the binding protein A27 is reduced. This assay also reflects the high potential of multivalent 2D inhibitors which is presumably attributable to their high binding affinity. The importance of sulfate residues for specific binding was shown since TRGO-dPG, lacking sulfate residues, did not show any significant binding.

### Inhibition of orthopoxvirus infection in vitro by carbon-based dPGS functionalized architectures

A cytotoxicity assay was performed on two cell lines (Hep2 and VeroE6) which were used in further infection experiments to rule out any influence of the used compounds on cell growth and viability. The compounds showed only low cytotoxicity up to a concentration of 125 µg mL⁻¹ in both cell lines. Consequently, 125 µg mL⁻¹ was used as the highest test concentration in the infection assay.

For the infection assay (PRNT) TRGO-dPG_{0.8}S_{0.5} was used, since it showed the best antibody inhibition results in the ELISA-based experiments. Heparin was used as a control since it has shown the best inhibition in the SPR experiment and the inhibitory effect with different virus strains and different cell lines is already well documented.

For the reference strain VACV IHD-W additionally lower concentrations of heparin were investigated. The results revealed that free heparin and the designed multivalent 2D architecture TRGO-dPG_{0.8}S_{0.5} successfully inhibit viral infection with the three poxvirus strains tested (VACV IHD-W, CPXV BR, and VACV WR) in Hep2.

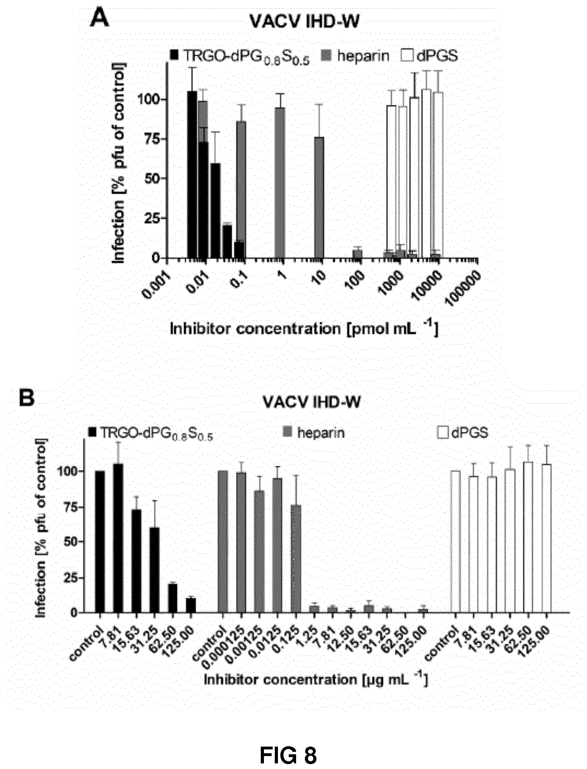

The results regarding VACV IHD-W are shown in Figures 8A and 8B depicting inhibition of virus infection by TRGO-dPG_{0.8}S_{0.5} in Hep2 cells in a PRNT using Vaccinia Virus International Health Department (VACV IHD-W: n=3, ±SD). Virus was pre-incubated with TRGO-dPG_{0.8}S_{0.5}, heparin, and free dPGS in serial dilutions and then added to the cells. Plaques were counted four days after infection. Concentrations are given in Figure 8A in pmol per mL and in Figure 8B in µg per mL.

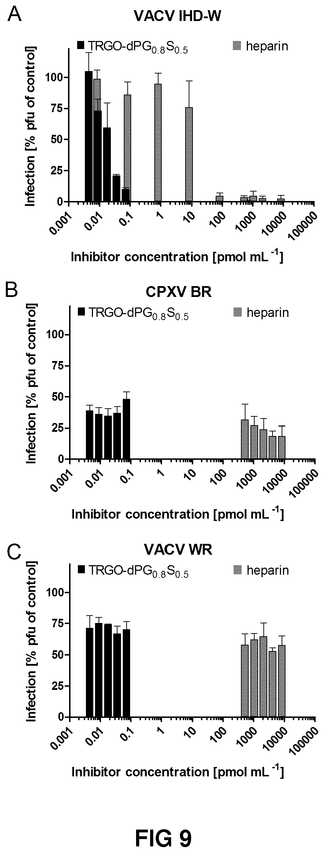

Parts of the results depicted in Figures 8A and 8B are also shown in Figure 9A. Further results of these experiments are shown in Figures 9B and 9C. Figures 9A to 9C depict inhibition of virus infection by TRGO-dPG_{0.8}S_{0.5} in Hep2 cells, wherein Figure 9A relates to Vaccinia Virus International Health Department (VACV IHD-W: n=3, ±SD) strain, Figure 9B relates to Cowpox Virus Brighton Red (CPXV B: n=4, ±SD) strain, and Figure 9C relates to Vaccinia Virus Western Reserve (VACV WR: n=2, ±SD) strain. All viruses were pre-incubated with TRGO-dPG_{0.8}S_{0.5} and heparin in serial dilutions for 30 minutes and then added to the cells and incubated for one hour. Plaques were counted four days after infection. Concentrations are given in pmol per mL.

TRGO-dPG_{0.8}S_{0.5} inhibits VACV IHD-W infection at the highest concentration tested (125 µg mL⁻¹) by approximately 90%, CPXV BR by approximately 60%, and VACV WR by approximately 30%. The same trend was observed when using Vero E6 cells. The different inhibition efficiencies for the three different virus strains in different cell lines can be explained by the fact that some orthopoxvirus strains enter cells additionally in a heparan sulfate-independent manner, and also by the variable level of expression of heparan sulfate in different cell lines.

Heparin, however, seems to be able to interfere with cell attachment of native virus but not with antibody binding to the A27 protein as seen in ELISA (cf. Figure 7A). For this observation we propose two explanations: First, the A27 protein is expressed multimerically on mature virions. Heparin and dPGS might not be able to effectively shield all binding sides for the specific antibodies. Secondly, both antibodies used in the sandwich ELISA show high avidity towards their binding domains on A27 and might therefore be able to displace heparin and dPGS in the ELISA assay. Moreover, antibodies only require specific target domains, whereas cell attachment is a much more complex process.

The fact that unbound dPGS caused no plaque reduction clearly indicates that free dPGS is obviously not able to cover heparan sulfate binding sites due to its small contact area and confirms the results of the SPR binding and inhibition studies. Furthermore, it underlines the importance of the newly synthesized multivalent 2D polymer architecture for efficient shielding of the orthopoxvirus particles.

The results prove a strong inhibitory effect of these novel multivalent 2D systems, and compared to heparin, dPGS, which has lower anticoagulant properties, exhibits a stronger binding to the poxviral surface protein A27. However, molecular masses differ greatly between the large multivalent architectures and heparin.

Looking at the effect on virus infection based on a molecular basis, it has to be considered that one TRGO-dPGS sheet represents approximately 125,000 heparin molecules. This implies that the newly designed multivalent 2D architecture can achieve the same reduction in plaque formation as heparin with at least 1,000 times higher efficacy per particle. Furthermore, the attachment to cell-associated heparan sulfate for viral entry is rather non-specifically used by other virus types, e.g. Herpes simplex virus (HSV), Dengue virus, or Cytomegalovirus (CMV). For these virus types the multivalent 2D structures will likely also be applicable.

Based on the results presented above, a tunable post-modification of the synthesized multivalent architectures by sulfation was demonstrated. The negatively charged sulfate groups enabled specific interactions with viral membrane proteins of different members of the genus orthopoxvirus, as shown by a competitive binding assay and a PRNT. Furthermore, the degree of sulfation and functionalization correlated with the binding efficiency, which could be explained by the multivalent effect of the multifunctional architecture. In comparison to linear heparin and depending on the virus strain investigated, these new multivalent 2D architectures showed an efficient inhibition of orthopoxvirus infections. In future, the binding efficiency of the system could be further increased not only by varying the degree of polymer functionalization or sulfation, but also by using different sizes of the flexible carbon sheets as well as types of post-modification. Overall, these new multivalent 2D polymer architectures have a high potential to inhibit various types of pox viruses as already demonstrated for the three tested poxvirus strains. They will also be interesting inhibitors for other known heparin sulfate binding viruses.

The tested compounds were produced as described above. The cell and virus cultivation as well as the explained assays were performed as will be described in the following.

### Cell and Virus cultivation

Vero E6 (ECACC 85020206) and Hep2 (ECACC86030501) were maintained using Dulbeccos's Modified Eagle Medium (DMEM) containing 1% L- glutamine and 10% or 5% fetal bovine serum, respectively. Vaccinia Virus Western Reserve (VACV WR ATCC® VR1354™), Vaccinia Virus International Health Department (VACV IHD-W ATCC® VR1441™), Cowpox Virus Brighton Red (CPXV BR ATCC® VR302™), and Monkeypox Virus (MPXV; RKI isolate #400619) were propagated on Hep2 cells. Cells were infected with a multiplicity of infection (MOI) of 0.1 and virus was harvested when a cytopathic effect was visible (2-4 days). Cells were then lysed by freeze-thawing and vortexing and supernatant was taken after centrifugation. For ELISA screenings VACV WR was heat inactivated for 1 h at 60 °C. Native viruses were purified by ultracentrifugation via a sucrose cushion (36% sucrose). Pelleted virus particles were resuspended in 1 mM Tris, stored at -80 °C and sonicated prior to all experiments. Virus titers were determined on Vero E6 cells in plaque forming units per mL (pfu ml⁻¹). All cell lines and virus stocks were screened for the absence of mycoplasma contamination.

### dPGS coat enzyme linked lectin assay (ELLA)

Pierce™ NeutrAvidin™ Coated Plates coated with biotinylated dPGS, dPG (2 µg ml⁻¹ PBS), or as control only PBS, were incubated for 1 h with serial dilutions of native viruses VACV WR, VACV IHD-W, CPXV BR (ranging from 2*107 pfu mL⁻¹ to 20 pfu mL⁻¹). After a washing step, monoclonal anti A27 antibodies (A1/40 and A3/710) (500 ng ml⁻¹ each) were added for another hour, washed off, and detected with a HRP coupled goat anti mouse antibody (IgG-HRP). Then, TMB (3,3',5,5'-Tetramethylbenzidin) substrate SeramunBlau©slow was added and stopped after 15 min with 0.25 M sulfuric acid. Absorption was measured at 450 nm measurement wavelength and 620 nm reference wavelength. All graphs and calculations were performed after background subtraction using the GraphPad Prism 5 software. All experiments were repeated at least two times in duplicates.

### Surface plasmon resonance (SPR) measurements

To test whether A27 binds to heparan sulfate or dPGS coated on a surface, biotinylated dPGS and heparan sulfate were immobilized at 5 µg ml⁻¹ and 5 µL min⁻¹ on a Biotin CAPture sensorchip. Starting with the lowest concentration serial dilutions (50, 16.6, 5.6, 1.86, 0.61 µg ml⁻¹) of recombinant A27 were passed over the surface with a flow rate of 30 µL min⁻¹ and 120 s of association and 300 s of dissociation (single cycle measurements). 100 µg ml⁻¹ salmon sperm DNA was added to A27 to prevent unspecific binding with the surface of the chip. For the SPR inhibition experiments A27 was preincubated with dPGS and heparin (100 µg ml⁻¹ each) and additionally with mAb A1/40 and mAb A3/710 at 100 µg ml⁻¹ and passed over the surface of a heparin sulfate coated chip at 10 µL min⁻¹ for 120 s association and 120 s dissociation. Non-treated A27 served as a control. Background measurements were performed by parallel injection of analytes over a control surface and nonspecific binding curves were subtracted.

### Sandwich ELISA

Screening of different graphene derivatives for inhibition of virus binding to A27 specific antibodies was determined by an ELISA-based assay. In short, Maxisorb plates in 96-well format were coated with A1/40 monoclonal antibody (200 ng per well). Serial dilutions of the graphene or polymer derivatives were pre-incubated with virus solution (25000 pfu mL⁻¹) for 30 min at 37 °C before the mixture was added to the wells and incubated for another 2 h at 37 °C. TRGO-dPG, dPGS, heparin, only virus or buffer served as controls. For analysis, biotinylated A3/710 antibody was added to the wells for 1 h at 37°C and streptavidin linked poly-horseradish-peroxidase for another 30 min at 37 °C. During all incubation steps samples were horizontally shaken at 300 rpm. For the detection, SeramunBlau©slow substrate was added for 15 min. Absorption was determined at 450 nm measurement wavelength and 620 nm reference wavelength. The half maximal inhibitory concentration (IC₅₀) was determined by setting the only virus containing control to 100% and the buffer treated control to 0% using the log (inhibitor) vs. normalized response - variable slope equation of the GraphPad Prism 5 software. Experiments were repeated at least 3 times in duplicates.

### Cytotoxicity assay

Cytotoxicity of the graphene derivatives was evaluated on Hep2 and Vero E6 cells by the CellTiter 96 AQueous One Solution Cell Proliferation Assay (MTS) from Promega according to the manufactures instructions. In short, Vero E6 and Hep2 cells were seeded in a 96-well plate (7500 cells/well) and cultured over night at 37°C before adding the graphene derivatives in serial dilutions. Non-treated cells served as control. After 1 h incubation, cells were washed and incubated in new cell culture media for another day before adding the MTS solution. After 2 h absorbance was measured at a measurement wavelength of 490 nm and a reference wavelength of 630 nm. Experiments were repeated 3 times in triplicates. The cell viability was calculated by setting the non-treated control to 100% after subtracting the background using the GraphPad Prism 5 software.

### Plaque Reduction Neutralization Test (PRNT)

For the PRNT cells (Hep2 and Vero E6) were seeded 16 h prior to infection in 48-well multidish format. Serial dilutions of graphene derivatives in cell culture medium were incubated for 30 min at 37°C with 20 pfu mL⁻¹ orthopoxvirus per well. Confluent cell monolayers were infected in triplicates with the mixtures for 1 h at 37 °C. Virus incubated with heparin or free dPGS served as control. Inoculum was removed and cells were washed twice with PBS. Fresh medium was added and wells were overlaid with semi-solid medium containing 1.6% Carboxymethylcellulose. After 4 days cells were stained with naphthol blue black and plaques were counted. Plaque numbers were normalized setting the only virus containing control to 100% and the medium treated control to 0%.

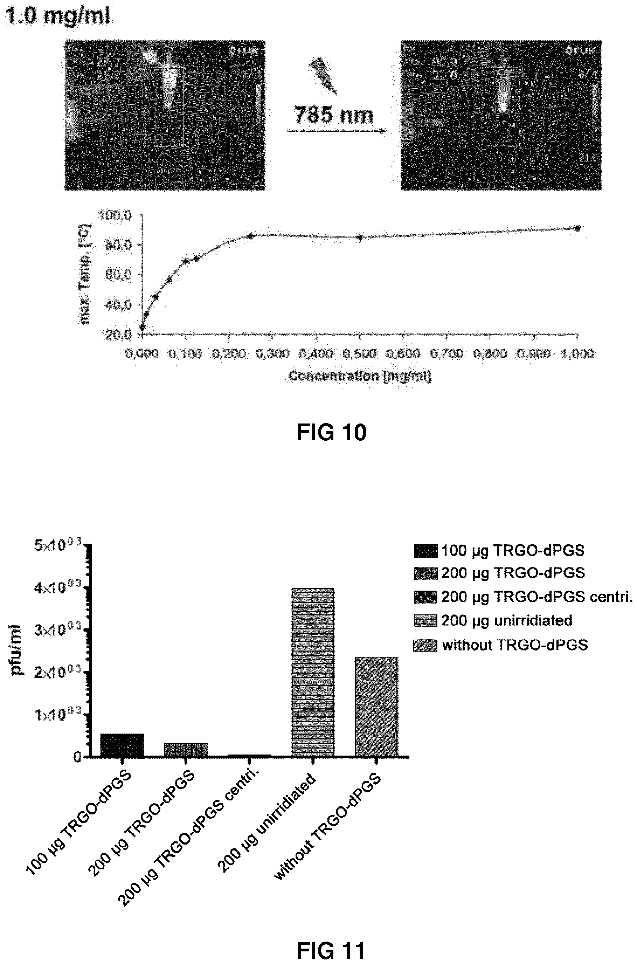

Figure 10 shows the results of an infrared irradiation of an exemplary embodiment of the sulfated graphene derivatives. TRGO-dPG_{0.8}S_{0.5} was subjected in different concentrations to infrared radiation having a wavelength of 785 nm. By an according infrared pulse, the temperature of TRGO-dPG_{0.8}S_{0.5} could be increased from approximately room temperature (between 21.8 and 27.7 °C) to more than 90 °C. This clearly shows the good heat absorption capability of dPGS-derivatized graphene compounds.

Figure 11 shows the results of an orthopoxvirus growth test bound to TRGO-dPG_{0.8}S_{0.5} after infrared irradiation at 785 nm. 100 µg or 200 µg TRGO-dPG_{0.8}S_{0.5} were mixed with 50 µl VACV IHD-W at a concentration of 5*10⁶ pfu/mL. Then, the mixture was subjected to infrared irradiation at 785 nm for 90 seconds. Afterwards, a plaque reduction neutralization test (PRNT) was carried out as explained above. As control, 200 µg of TRGO-dPG_{0.8}S_{0.5} were mixed with VACV IHD-W without subsequent infrared irradiation. As further control, VACV IHD-W were subjected to the same infrared irradiation without TRGO-dPG_{0.8}S_{0.5}.

The highest number of plaques could be observed in case of the sample that has not been subjected to infrared irradiation (fourth column). The second highest number of plaques could be observed in case of the sample that has not mixed with TRGO-dPG_{0.5}S_{0.5} (fifth column). Obviously, infrared light alone is not capable of destroying the orthopoxviruses.

In contrast, already at a concentration of 100 µg, TRGO-dPG_{0.8}S_{0.5} led to a significant decrease of formed plaques (first column). The heat absorbed by TRGO-dPG_{0.8}S_{0.5} due to the infrared irradiation resulted in a significant destruction of orthopoxviruses. This effect was even stronger if 200 µg TRGO-dPG_{0.8}S_{0.5} were used (second column).

The lowest number of formed plaques could be observed when centrifuging the mixture of 200 µg TRGO-dPG_{0.8}S_{0.5} and VACV IHD-W after infrared irradiation and determining the plaque formation in the obtained pellet. By this centrifugation, non-bound VACV IHD-W was removed from the sample so that essentially all remaining orthopoxviruses were subjected to a heat treatment due to strong infrared absorption of TRGO-dPG_{0.8}S_{0.5}.

The results depicted in Figure 11 clearly show that the claimed compounds can be well used for filtration and neutralization of pathogens from the air or from a liquid such as water (in vitro applications) or blood (in vivo or in vitro applications) These results also support the intended use of the claimed compounds in form of a semi-solid preparation (such as a gel, paste or cream) for the treatment of skin lesions, wherein a subsequent infrared irradiation of the such treated skin portions is to be performed using an appropriate wavelength (depending on absorption properties of the specific compound). Generally, graphene absorbs both infrared radiation and ultraviolet radiation. The skin lesions can be skin lesions originating from a herpes virus infection such as an infection by herpes simplex virus or by equine herpes virus.

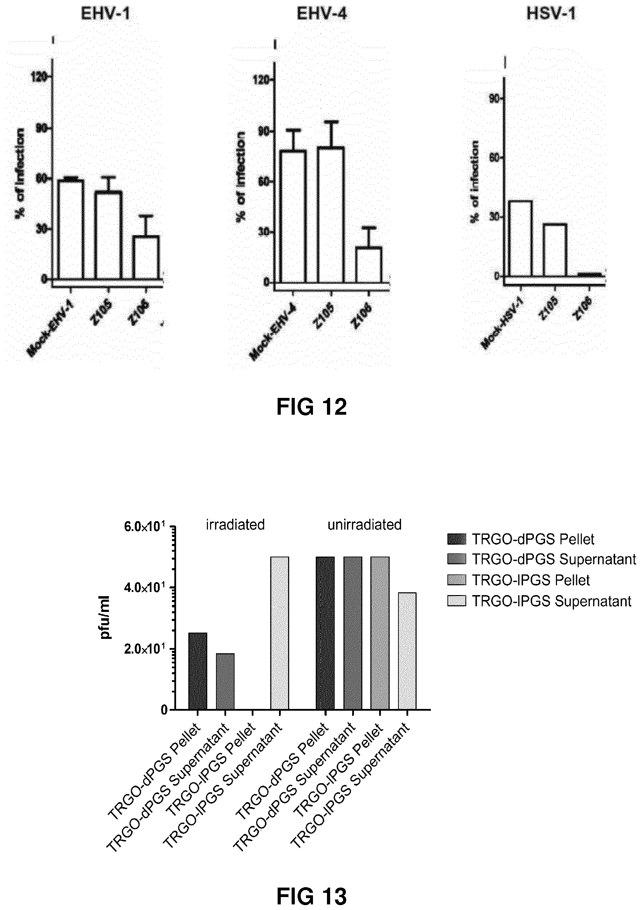

Figure 12 illustrates the capability of an exemplary embodiment of dPGS-derivatized graphene compounds to bind to viruses other than orthopoxviruses, namely to different herpes viruses, and to reduce the rate of infection of these viruses. EHV denotes equine herpes virus and HSV denotes herpes simplex virus. Mock-EHV-1, Mock-EHV-4 and Mock-HSV-1 denote negative controls (virus without dPGS-derivatized graphene). Z105 is a specific TRGO-dPG and Z106 denotes the sulfated analogue of Z105, i.e. a specific TRGO-dPGS.

The results were obtained by an infection assay in which the samples were examined 24 hours after infection by fluorescence activated cell sorting (FACS). The assay is similarly designed like the PRNT described above and can be summarized as follows:
First, 1 mg nanoparticles of dPGS-derivatized graphene are mixed with the respective virus. This mixture is then incubated for 1 hour at 37 °C and afterwards added to the cells to be tested. Finally, FACS counting takes place.

As can be seen from Figure 12, Z105 (TRGO-dPG) cannot reduce the rate of infection as compared to the negative controls in case of EHV. In case of HSV, the rate of infection is slightly decreased by TRGO-dPG. In contrast, Z106 (TRGO-dPGS) significantly reduces the rate of infection both in case of EHV and in case of HSV. Thereby, the binding of TRGO-dPGS to HSV-1 is very efficient so that the infectivity of HSV is almost completely destroyed. Thus, TRGO-dPGS is a class of very potent compounds for reducing the activity and/or infectivity of viruses, in particular of herpes viruses such as EHV and/or HSV in vivo or in vitro.

A further exemplary embodiment of a graphene derivative according to an aspect of the invention is a TRGO derivatized by a linear polyglycerol sulfate (TRGO-IPGS). The synthesis of these compounds can be carried out by a 1,3-dipolar cycloaddition according to Huisgen's reaction mechanism or by forming an aziridine under releasing nitrogen gas. This will be explained in the following in more detail.

### Preparation of TRGO-N-IPG

In this "graft to" process, TRGO (200 mg) was well dispersed in n-methyl-2- pyrrolidone (25 mL) under argon atmosphere in a 100 mL Schlenk flask by using a ultrasonic bath for 1 h. After this, the solubilized IPG-azide (one azide group) (950 mg in 5 mL NMP) was added to the black dispersion under continuous stirring and inert conditions. The reaction mixture was heated up to 160 °C and stirred for 72 h before cooling down to room temperature and diluting with distilled water (30 mL). For purification, the compound was centrifuged 5 times (6000 rpm, 15 min) before the precipitate was collected and dialyzed for 3 days in distilled water to remove the remaining n-methyl-2-pyrrolidone. A black solid was obtained after lyophilizing.

### Preparation of TRGO-N-IPGS

The TRGO-N-IPG (86 mg) was dispersed in p.a. DMF (10 mL) using an ultrasonic bath at 35 °C under inert conditions. After 30 min the stable dispersion was heated up to 60 °C under continuous stirring before a sulfur trioxide pyridine complex (420.0 mg, 2.2 mmol) dissolved in p.a. DMF (2 mL) under argon atmosphere was dropwise added over a period of 4 h. The dispersion was kept at 60 °C for 24 h before cooling down to room temperature. The reaction was quenched with distilled water (20 mL) and the pH was adjusted to 8 by addition of NaOH. The black dispersion was centrifuged 5 times (6000 rpm, 15 min) before the precipitate was collected and dialyzed for 3 days in distilled water to remove the remaining DMF. A black fluffy solid was obtained as product after drying in the lyophilizer.

The TRGO-IPGS prepared in this manner was inoculated with VACV IHD-W (concentration: 5*10⁶ pfu/ml) and afterwards irradiated with infrared light at a wavelength of 785 nm in the same way as explained with respect to Figure 11. TRGO-dPGS data is included in Figure 13 for comparison reasons. Non-irradiated samples served as negative control.

A good plaque formation could be observed in all negative controls that were not subjected to infrared irradiation. In addition, an equally high plaque formation was observed for TRGO-IPGS supernatant collected after centrifugation. The supernatant obviously contained VACV IHD-W that did not bind to TRGO-IPGS and that were thus not affected by the thermal treatment due to infrared irradiation.

In contrast, plaque formation was significantly decreased in case of TRGO-dPGS both in the pellet fraction and in the supernatant fraction after centrifugation. No plaque formation at all could be observed in the TRGO-IPGS fraction, indicating a very good binding of VACV IHD-W to TRGO-IPGS and a very efficient thermal inactivation of VACV IHD-W due to infrared irradiation of the complex of TRGO-IPGS and VACV IHD-W. Thus, TRGO-IPGS is a class of very potent compounds for reducing the activity and/or infectivity of viruses, in particular of orthopoxviruses such as VACV IHD-W.

### List of references cited in the preceding sections:

[1] R. R. Razonable, Mayo Clin Proc. 2011, 86, 1009-1026.
[2] L. Roden, D. S. Feingold, Trends in Biochem. Sci. 1985, 10, 407-409; C. H. Best, Circulation 1959, 19, 79.
[3] I. Capila, R. J. Linhardt, Angew. Chem. Int. Ed. 2002, 41, 390-412.
[4] Brodie, B. (1859) On the atomic weight of graphite, Phil. Trans. 149, 249-259.
[5] Hummers, Jr., W. S., and Offeman, R. E. (1958) Preparation of graphitic oxide, J. Am. Chem. Soc. 80, 1339.
[6] Tölle, F. J., Fabritius, M., and Mülhaupt, R. (2012) Emulsifier-free graphene dispersions with high graphene content for printed electronics and freestanding graphene films, Adv. Funct. Mater 22, 1136-1144.
[7] Appel, A.-K., Thomann, R., and Mülhaupt, R. (2012) Polyurethane nanocomposites prepared from solvent-free stable dispersions of functionalized graphene nanosheets in polyols, Polymer 53, 4931-4939.
[8] Roller, S., Zhou, H., and Haag, R. (2005) High-loading polyglycerol supported reagents for Mitsunobu- and acylation-reactions and other useful polyglycerol derivatives, Mol. Divers. 9, 305-316.
[9] Zhao, L., Takimoto, T., Ito, M., Kitagawa, N., Kimura, T., and Komatsu, N. (2011) Chromatographic separation of highly soluble diamond nanoparticles prepared by polyglycerol grafting, Angew. Chem. Int. Ed. 50, 1388-1392.
[10] Zhao, L., Chano, T., Morikawa, S., Saito, Y., Shiino, A., Shimizu, S., Maeda, T., Irie, T., Aonuma, S., Okabe, H., Kimura, T., Inubushi, T., and Komatsu, N. (2012) Hyperbranched polyglycerol-grafted superparamagnetic iron oxide nanorparticles: synthesis, characterization, functionalization, size seperation, magnetic properties and biological applications, Adv. Funct. Mater. 22, 5107-5117.
[11] Strom, T. A., Dillon, E. P., Hamilton, C. E., and Barron, A. R. (2010) Nitrene addition to exfoliated graphene: a one-step route to highly functionalized graphene, Chem. Commun. 46, 4097-4099.
[12] Liu, L.-H., Lerner, M. M., and Yan, M. (2010) Derivitization of pristine graphene with well-defined chemical functionalities, Nano Lett. 10, 3754-3756.
[13] Vadukumpully, S., Gupta, J., Zhang, Y., Xu, G. Q., and Valiyaveettil, S. (2011) Functionalization of surfactant wrapped graphene nanosheets with alkylazides for enhanced dispersibility, Nanoscale 3, 303-308.
[14] Sunder, A., Hanselmann, R., Frey, H., and Mülhaupt, R. (1999) Controlled synthesis of hyperbranched polyglycerols by ring-opening multibranching polymerization, Macromolecules 32, 4240-4246.
[15] A. Sunder, R. Mühlhaupt, R. Haag, H. Frey, Adv. Mater. 2000, 12, 235
[16] McAllister, M. J., Li, J.-L., Adamson, D. H., Schniepp, H. C., Abdala, A. A., Liu, J., Herrera-Alonso, M., Milius, D. L., Car, R., Prod'homme, R. K., and Aksay, I. A. (2007) Single sheet functionalized graphene by oxidation and thermal expansion of graphite, Chem. Mater. 19, 4396-4404.
[17] Dernedde, J., Rausch, A., Weinhart, M., Enders, S., Tauber, R., Licha, K., Schirner, M., Zügel, U., von Bonin, A., and Haag, R. PNAS 2010, 107, 46, 19679-19684.
[18] WO 2008/015015 A2 Dendritic polyglycerol sulfates and sulfonates and their use for inflammatory diseases

## Claims

1. Graphene derivative, comprising at least one graphene layer, **characterized in that** the graphene layer is functionalized with a covalently bound polyglycerol compound comprising a polyglycerol backbone and at least one substituent in the nature of a covalently bound sulfate group.

2. Graphene derivative according to claim 1, **characterized in that** the polyglycerol backbone has a degree of substitution between 10 and 100 %.

3. Graphene derivative according to any of the preceding claims, **characterized in that** the graphene layer is additionally substituted by hydroxyl groups.

4. Graphene derivative according to any of the preceding claims, **characterized in that** the polyglycerol has a dendritic backbone, in particular a hyperbranched backbone, or a linear backbone.

5. Use of a graphene derivative according to any of the preceding claims for binding viruses in vitro.

6. Graphene derivative according to any of claims 1 to 4 for use as a medicament.

7. Graphene derivative according to any of claims 1 to 4 for use according to claim 6, **characterized in that** the medicament is a medicament against viral diseases.

8. Antiviral medicament, comprising a graphene derivative according to any of claims 1 to 4.

9. Graphene derivative according to any of claims 1 to 4 for use as a diagnostic tool for the detection of viruses.

10. Use of a graphene derivative according to any of claims 1 to 4 as a diagnostic tool for the detection of viruses in vitro.

11. Use of a graphene derivative according to any of claims 1 to 4 as filtration system for filtrating viruses in vitro.

12. Graphene derivative according to any of claims 1 to 4 for use as filtration system for filtrating viruses in vivo.

13. Use of a graphene derivative according to any of claims 1 to 4 as medical device against viruses in vitro.

14. Graphene derivative according to any of claims 1 to 4 for use as medical device against viruses in vivo.

15. Method for manufacturing a graphene derivative according to any of claims 1 to 4, **characterized by** the following steps:
a) providing at least one graphene layer substituted by hydroxyl groups,
b) mixing the substituted graphene layer with glycidol,
c) reacting the gylcidol on the graphene layer to covalently bound polyglycerol,
d) modifying the covalently bound polyglycerol with a sulfate group by adding a sulfation reagent.

16. Method for manufacturing a graphene derivative according to any of claims 1 to 4, **characterized by** the following steps:
a) providing at least one graphene layer substituted by functional groups,
b) mixing the substituted graphene layer with a grafting compound to achieve a covalent bond between the polyglycerol and the graphene,
c) modifying the covalently bound polyglycerol with a sulfate group by adding a sulfation reagent.

## Patentansprüche

1. Graphenderivat, umfassend wenigstens eine Graphenschicht, **dadurch gekennzeichnet, dass** die Graphenschicht mit einer kovalent gebundenen Polyglycerolverbindung, die ein Polyglycerolgerüst und wenigstens einen Substituenten mit der Beschaffenheit einer kovalent gebundenen Sulfatgruppe umfasst, funktionalisiert ist.

2. Graphenderivat gemäß Anspruch 1, **dadurch gekennzeichnet, dass** das Polyglycerolgerüst einen Substitutionsgrad von zwischen 10 und 100 % aufweist.

3. Graphenderivat gemäß einem der vorstehenden Ansprüche, **dadurch gekennzeichnet, dass** die Graphenschicht zusätzlich mit Hydroxygruppen substituiert ist.

4. Graphenderivat gemäß einem der vorstehenden Ansprüche, **dadurch gekennzeichnet, dass** das Polyglycerol ein dendritisches Gerüst, insbesondere ein hyperverzweigtes Gerüst, oder ein lineares Gerüst aufweist.

5. Verwendung eines Graphenderivats gemäß einem der vorstehenden Ansprüche zum Binden von Viren *in vitro.*

6. Graphenderivat gemäß einem der Ansprüche 1 bis 4 zur Verwendung als Medikament.

7. Graphenderivat gemäß einem der Ansprüche 1 bis 4 zur Verwendung gemäß Anspruch 6, **dadurch gekennzeichnet, dass** das Medikament ein Medikament gegen Viruserkrankungen ist.

8. Antivirales Medikament, umfassend ein Graphenderivat gemäß einem der Ansprüche 1 bis 4.

9. Graphenderivat gemäß einem der Ansprüche 1 bis 4 zur Verwendung als diagnostisches Werkzeug zum Nachweisen von Viren.

10. Verwendung eines Graphenderivats gemäß einem der Ansprüche 1 bis 4 als diagnostisches Werkzeug zum Nachweisen von Viren *in vitro.*

11. Verwendung eines Graphenderivats gemäß einem der Ansprüche 1 bis 4 als Filtrationssystem zum Filtrieren von Viren *in vitro.*

12. Graphenderivat gemäß einem der Ansprüche 1 bis 4 zur Verwendung als Filtrationssystem zum Filtrieren von Viren *in vivo.*

13. Verwendung eines Graphenderivats gemäß einem der Ansprüche 1 bis 4 als medizinische Vorrichtung gegen Viren *in vitro.*

14. Graphenderivat gemäß einem der Ansprüche 1 bis 4 zur Verwendung als medizinische Vorrichtung gegen Viren *in vivo.*

15. Verfahren zur Herstellung eines Graphenderivats gemäß einem der Ansprüche 1 bis 4, **gekennzeichnet durch** die folgenden Schritte:
a) Bereitstellen wenigstens einer mit Hydroxygruppen substituierten Graphenschicht,
b) Mischen der substituierten Graphenschicht mit Glycidol,
c) Umsetzen des Glycidols an der Graphenschicht zu kovalent gebundenem Polyglycerol,
d) Modifizieren des kovalent gebundenen Polyglycerols mit einer Sulfatgruppe durch Zugeben eines Sulfatierungsreagens.

16. Verfahren zur Herstellung eines Graphenderivats gemäß einem der Ansprüche 1 bis 4, **gekennzeichnet durch** die folgenden Schritte:
a) Bereitstellen wenigstens einer mit funktionellen Gruppen substituierten Graphenschicht,
b) Mischen der substituierten Graphenschicht mit einer Pfropfverbindung, um eine kovalente Bindung zwischen dem Polyglycerol und dem Graphen zu erzeugen,
c) Modifizieren des kovalent gebundenen Polyglycerols mit einer Sulfatgruppe durch Zugeben eines Sulfatierungsreagens.

## Revendications

1. Dérivé de graphène, comprenant au moins une couche de graphène, **caractérisé en ce que** la couche de graphène est fonctionnalisée avec un composé de polyglycérol lié de façon covalente comprenant un squelette de polyglycérol et au moins un substituant de type groupe sulfate lié de façon covalente.

2. Dérivé de graphène selon la revendication 1, **caractérisé en ce que** le squelette de polyglycérol a un degré de substitution de 10 à 100 %.

3. Dérivé de graphène selon l'une quelconque des revendications précédentes, **caractérisé en ce que** la couche de graphène a en outre subi une substitution par des groupes hydroxyle.

4. Dérivé de graphène selon l'une quelconque des revendications précédentes, **caractérisé en ce que** le polyglycérol a un squelette dendritique, en particulier un squelette hyper ramifié, ou un squelette linéaire.

5. Utilisation d'un dérivé de graphène selon l'une quelconque des revendications précédentes pour la liaison de virus *in vitro.*

6. Dérivé de graphène selon l'une quelconque des revendications 1 à 4, pour utilisation comme médicament.

7. Dérivé de graphène selon l'une quelconque des revendications 1 à 4, pour utilisation selon la revendication 6, **caractérisé en ce que** le médicament est un médicament contre les maladies virales.

8. Médicament antiviral, comprenant un dérivé de graphène selon l'une quelconque des revendications 1 à 4.

9. Dérivé de graphène selon l'une quelconque des revendications 1 à 4, pour utilisation comme outil de diagnostic pour la détection de virus.

10. Utilisation d'un dérivé de graphène selon l'une quelconque des revendications 1 à 4 comme outil de diagnostic pour la détection de virus *in vitro.*

11. Utilisation d'un dérivé de graphène selon l'une quelconque des revendications 1 à 4 comme système de filtration pour filtrer des virus *in vitro.*

12. Dérivé de graphène selon l'une quelconque des revendications 1 à 4, pour utilisation comme système de filtration pour filtrer des virus *in vivo.*

13. Utilisation d'un dérivé de graphène selon l'une quelconque des revendications 1 à 4 comme dispositif médical contre des virus *in vitro.*

14. Dérivé de graphène selon l'une quelconque des revendications 1 à 4, pour utilisation comme dispositif médical contre des virus *in vivo.*

15. Procédé de fabrication d'un dérivé de graphène selon l'une quelconque des revendications 1 à 4, **caractérisé par** les étapes suivantes :
a) la fourniture d'au moins une couche de graphène ayant subi une substitution par des groupes hydroxyle,
b) le mélange de la couche de graphène ayant subi une substitution avec du glycidol,
c) la réaction du glycidol présent sur la couche de graphène avec un polyglycérol lié de façon covalente,
d) la modification du polyglycérol lié de façon covalente avec un groupe sulfate en ajoutant un réactif de sulfatation.

16. Procédé de fabrication d'un dérivé de graphène selon l'une quelconque des revendications 1 à 4, **caractérisé par** les étapes suivantes :
a) la fourniture d'au moins une couche de graphène ayant subi une substitution par des groupes fonctionnels,
b) le mélange de la couche de graphène ayant subi une substitution avec un composé de greffage pour obtenir une liaison covalente entre le polyglycérol et le graphène,
c) la modification du polyglycérol lié de façon covalente avec un groupe sulfate en ajoutant un réactif de sulfatation.
